# EUROPEAN PATENT APPLICATION

(11) **EP 4 056 053 A1**
(43) Date of publication of application: **14.09.2022**
(21) Application number: 21162451.5
(22) Date of filing: 12.03.2021
(51) Int. Cl.: A23L 33/135, C12N 1/20

(54) **PRE-CONDITIONING OF L. REUTERI**

(71) Applicant: Biogaia AB, 112 27 Stockholm (SE)
(72) Inventor: ROOS, Stefan, 757 54 Uppsala (SE); HORCAJADA, Marie Noëlle, 01170 ECHENEVEX (FR); BONNET, Nicolas, 74380 NANGY (FR); SABATIER, Magalie, 1000 LAUSANNE 26 (CH); BOURQUI, Bertrand, 3013 BERN (CH); PRIOULT, Guénolée Eliane Marie, 3007 BERN (CH); DE BRUYN, Florac, 1006 LAUSANNE (CH)
(74) Representative: Barker Brettell Sweden AB

(57) **Abstract**

The present invention relates to probiotic *Lactobacillus reuteri* strain obtained by a step of growing the bacteria in a medium comprising galacto-oligosaccharides (GOS), so called pre-conditioning. The pre-conditioning method provides boosting effects of the probiotic *L. reuteri* strain, such as improved mineral absorption, in the gastrointestinal tract. The invention comprises methods for manufacturing and cultivating probiotic *Lactobacillus reuteri* strains, and products and first and second medical uses containing such strains.

## Description

### TECHNICAL FIELD

The invention herein relates generally to enhancing the survival, persistence and/or probiotic effect of probiotic *Lactobacillus reuteri* strains in mammals or birds. The invention comprises methods for manufacturing and cultivating probiotic *L. reuteri* strains, and products containing such strains. In more detail, the present invention relates to a probiotic *L. reuteri* strain obtained by a step of growing the bacteria in a growth medium comprising galacto-oligosaccharides (GOS), thereby enhancing the survival and persistence and/or boosting beneficial probiotic effects of the probiotic *L. reuteri* strains, such as improved mineral absorption, improved protective effect of the epithelial integrity in the gastrointestinal tract and improved bone formation and mineralization effects. Moreover, this invention relates to preparations comprising substrate components being specifically selected to enhance the survival, persistence and/or probiotic effect of such probiotic *L. reuteri* strains for certain circumstances.

### BACKGROUND

Probiotics are defined as "live microorganisms that, when administered in adequate amounts, confer a health benefit on the host." This is the widely accepted scientific definition around the world (see e.g., Hill et al. 2014; Nat. Rev. Gastro. & Hepathology, Vol 11). Probiotic products (which is usually dietary supplements or foods) may be recommended for different conditions or symptoms an individual is experiencing. Lactic acid producing bacteria, such as lactobacilli, are commonly used as probiotics in various types of foods, for example yoghurt. Growth and colonization of harmful microorganisms can be prevented by such lactic acid producing bacteria through their own colonization inside the intestinal system, through competition of available nutrients and/or through production of specific substances, such as hydrogen peroxide, bacteriocins and/or organic acids, including lactic and acetic acid, that lowers the intestinal pH. It is well established that interactions between host and gut microbes are fundamental to health and well-being of the host. Intestinal microbiota generates metabolites that provide the host with nutrients but may also be involved in the immune response and in regulation and development of the host's immune system as well as reducing inflammation and preventing allergic responses.

Prebiotics are compounds that induce the growth or activity of beneficial microorganisms, such as bacteria and fungi, by selectively stimulating their growth and/or activity. In the gastrointestinal tract, prebiotics can therefore alter the composition of the gut microbiome. Dietary prebiotics are typically nondigestible fiber compounds that pass undigested through the upper part of the gastrointestinal tract and stimulate the growth or activity of advantageous bacteria that colonize the large bowel by acting as their substrate. Various compounds have been tested to determine their function as prebiotics. Fructo-oligosaccharides (FOS), galacto-oligosaccharides (GOS), and trans-galacto-oligosaccharides (TOS) are the most common prebiotics. Consuming certain prebiotics can, thus, improve immunity functions by increasing the population of microorganisms associated with human health, and animal and human studies have shown that prebiotics can decrease the population of harmful bacteria. Prebiotics are, thus, ingredients, providing health benefits when fermented by the native microflora in the intestine of a subject or by probiotic bacteria ingested simultaneously with the prebiotics.

Combining particular types of prebiotics and probiotics is generally known to give rise to certain synergistic effects. In particular, the effect of combined prebiotic GOS and probiotic microorganisms, such as lactobacilli and bifidobacteria, has been documented (see e.g. YUTAKA KANAMORI, MD et al., Digestive Diseases and Sciences, Vol. 46, No. 9 (September 2001), pp. 2010-2016 (^{©} 2001)). It has been demonstrated that the synbiotic effect between prebiotics and probiotics is due to the consumption of the prebiotic by the probiotic within the gastrointestinal (GI) tract of the subject.

The manufacturing procedure of probiotic lactic acid producing bacteria is typically standardized and involves a step of fermenting the bacteria in a growth medium comprising a carbohydrate source, such as a sugar, for example glucose, fructose, sucrose, lactose or dextrose. Following the fermentation, the probiotic bacteria are usually cryoprotected and frozen or freeze-dried and packaged into a finished product.

The ingestion of probiotic lactic acid bacteria has various effects *in vivo.* One such important effect is the bioavailability of essential minerals, which can be significantly influenced by the presence of such probiotic lactic acid bacteria. Although the content of minerals in the body depends primarily on their supply from food intake and they are absorbed mainly in the GI tract, mere provision of minerals via food intake does not necessarily lead to a sufficient mineral bioavailability. The main factors affecting mineral bioavailability are the content of minerals in the food, synergistic and antagonistic interactions between minerals in the food and in the GI tract, the presence of complexing or chelating compounds in the food, and the health state of the organism and its age. Also, the intestinal microflora, probiotics and prebiotics significantly influence the bioavailability of minerals and can thereby increase or decrease the absorption of such minerals.

Minerals are essential for all living species, even though the specific requirements differ between species. Minerals have a great number of important functions in the human organism and include e.g., iron, calcium and magnesium, among many others. Mineral deficiencies, i.e., low or suboptimal amounts of such minerals in the body, typically by ingesting minerals below recommended RDA amounts, can lead to diseases and so can mineral excess. It is therefore pivotal to obtain the correct amounts of minerals at the correct ratios for optimal health. Most natural diets will provide these minerals in appropriate balances, but there are situations when this is not enough to maintain health.

Calcium for example, is the most abundant mineral in the human body and is an important bone component and an enzyme activator. Calcium also takes part in conduction of bioelectric impulses, blood coagulation, muscle contraction, inflammation and hormonal secretion. Calcium is absorbed in the small intestine in the presence of the active form of vitamin D (calcitriol). Hypocalcemia, commonly known as calcium deficiency, can lead to various side effects, conditions or even severe diseases. Calcium deficiency is most common in older adults, teenagers, and people who are overweight. The reported prevalence for permanent hypocalcemia ranges from 0.4% and 33% with renal failure remaining the most common type of hypocalcemia, followed by vitamin D deficiency. While the incidence of hypocalcemia is difficult to quantify, global estimates in accounted for 3.4 billion people at risk for calcium deficiency. Usually, average calcium levels are kept through the actions of the parathyroid hormone (PTH), the kidneys, and the intestines. Calcium deficiency can lead to various conditions or disorders, such as muscle problems, e.g., cramps, muscle spasms, and aches, to fatigue, including insomnia, sleepiness, and extreme fatigue, to skin and nail symptoms, such as eczema, to psoriasis, redness, itchiness, and skin blisters, osteoporosis and osteopenia, to painful premenstrual syndrome, dental problems, depression, etc. Populations at risk for calcium deficiency, include particularly pregnant women (especially in the last trimester), lactating women, postmenopausal women, and, possibly, elderly men. However, calcium deficiency (hypocalcemia) is also serious problem in early infancy. Hypocalcemia in early infancy is usually termed neonatal hypocalcemia. Neonatal hypocalcemia particularly occurs in the first 2 to 3 days of a baby's life. To some degree neonatal hypocalcemia is part of a normal developmental process and can be efficiently treated by normal nutritional intervention. A late hypocalcemia starts in the first week or weeks after birth and is less likely to go away without nutrition support and can be efficiently treated by corresponding supplements or fortified infant formulae. Notably, low magnesium levels may also cause low calcium levels, since calcium levels are linked to a certain extent to levels of magnesium.

Magnesium is a bone component and takes part in neuronal conduction and ribosomal processes. It also helps to maintain the health of muscles and it plays a role in over 300 enzymatic reactions in the body. It is absorbed in the small intestine. Low levels of magnesium or magnesium deficiency (hypomagnesemia) may result *inter alia* from gastrointestinal causes, such as inadequate bioavailability and absorption of magnesium in the small intestine. Magnesium deficiency may also result from kidney causes, poor intake of magnesium due to eating behavior or diets, or the concurrent use of certain medications, e.g., administration of proton-pump inhibitors, etc. Deficiency of magnesium can cause tiredness, generalized weakness, muscle cramps, abnormal heart rhythms, increased irritability of the nervous system with tremors, paresthesia, palpitations, low potassium levels in the blood, hypoparathyroidism which might result in low calcium levels in the blood, chondrocalcinosis, spasticity and tetany, migraines, epileptic seizures, basal ganglia calcifications and in extreme and prolonged cases coma, intellectual disability or death. Magnesium also plays an important role in carbohydrate metabolism and its deficiency may worsen insulin resistance, a condition that often precedes diabetes, or may be a consequence of insulin resistance. Populations at risk for hypomagnesemia, include particularly diabetes patients, patients at risk of diabetes, obese patients, overweight patients, etc.

Iron for instance is absorbed in the duodenum and the small intestine in the form of Fe²⁺. Iron is a component of hemoglobin, which is responsible for the transport of oxygen, and myoglobin, and it is also a part of many enzymes such as catalase, peroxidase and cytochromes. In addition, iron is very important in maintaining several other body functions, such as in the maintenance of healthy cells, skin, hair, and nails. Cells that line the gastrointestinal membranes absorb iron from the ingested food. Iron can be stored in the liver and released as needed to make new red blood cells in the bone marrow. Iron deficiency is a condition which occurs when the body doesn't have enough of the mineral iron and often this leads to abnormally low levels of red blood cells and a condition referred to as iron deficiency anemia. As a result, iron deficiency anemia may leave you tired and short of breath. Iron deficiency can be caused by blood loss, lack of dietary iron, an inability to absorb iron, and during pregnancy. Several risk factors and risk groups are associated with iron deficiency anemia such as women with heavy menstruation or pregnancy. Another group at risk for iron deficiency is infants, especially those with a low birth weight or which are born prematurely, or those infants who don't get enough iron from breast milk or formula. In general, children need extra iron during growth spurts. Furthermore, a poor diet or certain intestinal diseases that affect how the body absorbs iron can also cause and be a risk factor for iron deficiency anemia. Iron deficiency is thus very common in certain groups of people and even if it, in most cases, does not lead to any severe complications; untreated iron deficiency can have detrimental effects. Taken together, there is a need and room for new methods and safe products that can increase the iron levels in patients with iron deficiency and that can be used in treatment of an iron deficiency and diseases or disorders related to iron deficiency.

Iron and iron levels are of particular importance in pregnant subjects, fetus and babies/infants. Infants and toddlers are vulnerable to iron deficiencies, especially in developing countries, due to the high dietary requirement in the weaning period in proportion to body size. During this period, iron is a critical nutrient for development, particularly for brain and muscle tissues. Prevalence of iron deficiency is the highest at 1 to 3 years of age. The prevalence in Europe ranges between 5 and 20% (Domellöf, M et al, D. (2014)), and can be higher for Asian countries, up to 35% (Duggan, MB et al, S. (1991)).

Recent studies have produced new information on microbiota - mineral interactions and have mainly concerned the impact of intestinal bacteria on the metabolism of calcium and iron. Given the aging population and the rising prevalence of osteoporosis among postmenopausal women and men, the new data on calcium metabolism and hence on bone metabolism and its improvement are crucial to the development of the field. In contrast, the results of research into adverse interactions between iron and the intestinal microflora show that there is a crucial need for further studies on the bioavailability of this mineral in order to prepare new recommendations regarding iron supplementation.

Also, a common problem with oral administration of probiotic bacteria is that they arrive at their destined location in the gastrointestinal tract in insufficient amounts and/or that the activity of the probiotic bacteria in these locations of the intestinal tract, where they assert their effects, is inadequate. The survival, viability and engraftment of the probiotic bacteria in the gastrointestinal tract is thus a significant challenge for those who manufacture probiotic products. As a consequence, the dosage of probiotic bacteria has to be increased and/or more frequent administration is required to obtain the desired probiotic effect. This further leads to problems with unnecessary costs, undesirable frequency of intake and possibly also decreased health benefits when the probiotic bacteria do not sufficiently provide the desired effects. Thus, it is desired to improve the probiotic effect without increasing the dosage.

### SUMMARY OF THE INVENTION

It is an object of the current invention to provide more efficient means, methods and therapies of improving the survival, persistence and/or probiotic effects of certain specific probiotic microorganisms, such as probiotic *L. reuteri* strains, when administering such probiotic *L. reuteri* strains to a subject or a patient in need thereof. Moreover, it is a particular object to prevent and/or treat mineral deficiency in a subject or in a patient in need thereof, particularly mineral deficiencies of minerals selected from magnesium, iron and/or calcium.

These objects are solved by the current invention as addressed below.

The present invention is defined below and in the independent claims. Further embodiments of the invention are defined in the specification and in the dependent claims.

In a first aspect, the invention provides a method for preparing a pre-conditioned probiotic *L. reuteri* strain characterized in that the method comprises the following steps:
a) cultivating a probiotic *L. reuteri* strain in the presence of GOS, thereby pre-conditioning the probiotic *L. reuteri* strain; and
b) harvesting the pre-conditioned probiotic *L. reuteri* strain from the growth medium.

Cultivating a probiotic *L. reuteri* strain in the presence of GOS in step a) of the inventive method leads to formation of the pre-conditioned *L. reuteri* strain.

In the inventive method, the method GOS may be added in step a) to the growth medium in an amount of at least 0.2 wt%, preferably at least 0.5 wt%, even more preferably at least 0.75 wt% of GOS in the growth medium, and preferably at most 8 wt% of GOS, such as at most 7 wt% GOS, at most 6 wt% GOS, or even at most 5 wt% GOS, more preferably at most 4 wt% of GOS or even at most 3% GOS, at most 5 wt% of GOS, preferably at most 4 wt% of GOS, e.g., at most 3 wt% of GOS, at most 2 wt% of GOS, or at most 1 wt% of GOS in the growth medium, preferably calculated on basis of the growth medium (% w/w). Any combination of these upper and lower ranges is encompassed herewith. More preferably, GOS is added to the growth medium in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium (% w/w). The GOS may be added in step a) at the start of cultivation of a probiotic *L. reuteri* strain, at once, stepwise or continuously during the cultivation step a) of the probiotic *L. reuteri* strain, but possibly also in the middle or at the end of cultivation step a). Moreover, the GOS added to the growth medium in step a) of the inventive method preferably may have a degree of polymerization (DP) of 3-8. The cultivation in step a) of the inventive method may occur over a period of 5 to 18 hours, preferably 7 to 14 hours, and more preferably 10 to 13 hours, and even more preferably about 12 hours. According to a preferred embodiment, cultivation in step a) of the inventive method occurs at a temperature of between 25 to 45°C, preferably between 30 and 40°C, most preferably at a temperature of about 37°C. The pH during pre-conditioning of probiotic *L. reuteri* strain during cultivation step a) may be either not controlled or set to a constant value throughout the cultivation step a), typically to be monitored and adjusted during cultivation step a). Accordingly, the pH may be between 3.0 and 7.0, particularly when pH is not controlled during pre-conditioning of probiotic *L. reuteri* strain. Alternatively, if pH is controlled during cultivation step a), pH may be adjusted to a range of about 5.0 to 7.0, e.g., about 5.0 to 6.0, about 5.5 to 6.5 (e.g., about 6.0±0.2) or about 6.0 to 7.0 (e.g., about 6.6±0.2). For instance, pH may be adjusted to a range of about 5.5 to 6.5, preferably pH may be adjusted to a range of about 6.0±0.4, and more preferably pH may be adjusted to a range of about 6.0±0.2. Alternatively, pH may be adjusted to a range of about 6.0 to 7.0, preferably about 6.6±0.4, and more preferably 6.6±0.2. It is added that the inventive method for preparing the pre-conditioned probiotic *L. reuteri* strain may comprises a further step c) of washing the pre-conditioned probiotic *L. reuteri* strain after harvesting step b), preferably to remove trace amounts of growth medium. The inventive method may also comprise a further step d) of drying the harvested pre-conditioned probiotic *L. reuteri* strain, step d) either carried out after washing step c) or after harvesting step b). The drying step may be carried out using spray-drying, fluid bed drying, air convective drying, atmospheric drying, roller drying or freeze drying, lyophilizing, and more preferably spray-drying or freeze drying. In case of need, the drying step may be preceded by treating the harvested pre-conditioned probiotic *L. reuteri* strain with a protectant, a cryoprotectant, a lyoprotectant and/or a carrier. Alternatively, or additionally, the pre-conditioned *L. reuteri* strain obtained after any of steps b), c) or d) may be encapsulated for further processing, preferably prior to the drying step. Particularly preferably, the probiotic *L. reuteri* strain is at least one *L. reuteri* strain or multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L*. *reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

According to a second aspect, the invention provides a composition comprising an effective amount of a pre-conditioned probiotic *L. reuteri* strain, wherein the pre-conditioned probiotic *L. reuteri* strain has been prepared by cultivating a *L. reuteri* strain in the presence of GOS, preferably according to a method or process as defined herein. The inventive composition may comprise the pre-conditioned probiotic *L. reuteri* strain in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose, or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose, more preferably around 10⁷ cfu to 10⁹ cfu per daily dose. A preferred daily dose is around 10⁸ total cfu's, e.g., 10⁷ to 10⁹ cfu per daily dose or 10⁸ to 10⁹ cfu per daily dose. As outlined herein, the pre-conditioning of the probiotic *L. reuteri* strain of the inventive composition preferably allows or is for improving or increasing the survival, persistence and/or probiotic effect, typically of the *L. reuteri* strain in the intestinal tract of a subject. In more detail, the pre-conditioning of the probiotic *L. reuteri* strain increases or improves the survival and/or persistence of the probiotic *L. reuteri* strain, which is manifested, among others, through an increase in stress tolerance of the *L. reuteri* strain, and/or an increase in adhesion to mucus of the probiotic *L. reuteri* strain. The inventive composition may likewise be used for increasing mineral bioavailability in the intestinal tract of a subject, for increasing iron and calcium solubility and/or absorption in the intestinal tract of a subject, and/or for increasing the protective effect of the epithelial integrity in the gastrointestinal tract and/or for promoting the bone formation and/or bone mineralization, and/or for promoting the maturation and/or activity of osteoblasts in a subject. The inventive composition may be furthermore a solid or liquid, and/or may be present in form of a powder, a tablet, a capsule, or may be in form of an oil formulation, an emulsion, an oil-in-water emulsion (o/w emulsion), or a water-in oil emulsion (w/o emulsion). Additionally, without being limiting thereto, the inventive composition may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

In a third aspect, the invention provides a non-therapeutic use of an effective amount of a pre-conditioned probiotic *L. reuteri* strain as prepared according to the invention or as described herein for increasing or boosting the survival, persistence and/or probiotic effect of the probiotic *L. reuteri* strain in the digestive tract of a healthy subject. In more detail, the pre-conditioning of the probiotic *L. reuteri* strain increases or improves the survival and/or persistence of the probiotic *L. reuteri* strain, which is manifested, among others, through an increase in stress tolerance of the *L. reuteri* strain, and/or an increase in adhesion to mucus of the probiotic *L. reuteri* strain. Accordingly, the probiotic effects of the probiotic *L. reuteri* strain are increased or improved by the pre-conditioning. This means that the pre-conditioning of the probiotic *L. reuteri* strain enables the probiotic *L. reuteri* strain to be used for increasing mineral bioavailability in the intestinal tract of a subject, for increasing iron and calcium solubility and/or absorption in the intestinal tract of a subject, and/or for increasing the protective effect of the epithelial integrity in the gastrointestinal tract, and/or for promoting the bone formation and/or bone mineralization, and/or for promoting the maturation and/or activity of osteoblasts in a subject. In the inventive non-therapeutic use the (thereby formed) composition (i.e., comprising the pre-conditioned probiotic *L. reuteri* strain) preferably increases mineral solubility, accessibility and/or absorption of minerals in the intestinal tract of the healthy subject, and/or increases survival and/or engraftment and/or viability of a probiotic *L. reuteri* strain in the intestinal tract of the healthy subject. Particularly relevant minerals in this context include magnesium, calcium and/or iron, preferably calcium and iron. Moreover, the healthy subject is typically selected from an athlete, a child, a toddler or an infant, an adult, an elderly, a pregnant woman, a vegetarian, a lactating woman, a companion animal, a cat, or a dog or a bird, such as poultry. A healthy subject in this context does usually not suffer from any disease or condition, particularly any diseases or condition related to mineral deficiency. The inventive composition may be solid or liquid, and/or is present in form of a powder, a tablet, a capsule, or may be in form of an oil formulation, an emulsion, an oil-in-water emulsion (o/w emulsion), or a water-in oil emulsion (w/o emulsion). Moreover, the inventive composition may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage. As before, the *L. reuteri* strain is particularly preferable at least one *L. reuteri* strain or of multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L*. *reuteri* strain as deposited under DSM 17938.

In one embodiment, the increase in survival and/or persistence of the *L. reuteri* leads to an increase in probiotic effect of the probiotic *L. reuteri* strain.

In one embodiment, the increase in probiotic effect of the probiotic *L. reuteri* strain is an increase in survival and/or persistence of the probiotic *L. reuteri* strain.

In one embodiment, the increase in probiotic effect of the probiotic *L. reuteri* strain is an increase in activity, such as in bioactivity of the *L. reuteri,* which may result in an increased effect on the surrounding environment, such as other gut microorganisms and/or the host cells. In more detail, these effects may be exemplified as an increase in mineral absorption, an increase in protective effect of the epithelial integrity, an increase in bone formation and/or bone mineralization, and/or an increase in maturation and/or activity of osteoblasts. In another embodiment the increase in probiotic effect of the *L. reuteri* is a promotion of a healthy microbiota.

According to a fourth aspect, the invention provides a composition comprising an effective amount of a pre-conditioned probiotic *L. reuteri* strain as prepared according to the invention or as described herein for use as a medicament.

According to a fifth aspect, the invention provides a composition comprising an effective amount of a pre-conditioned probiotic *L*. *reuteri* strain as prepared according to the invention or as defined herein for (therapeutic) use in the prevention and/or treatment of a disease in patient in need thereof.

In the above mentioned fourth and fifth aspect, the patient is preferably at risk of developing mineral deficiency or is a subject suffering from mineral deficiency.

Moreover, the pre-conditioned probiotic *L. reuteri* strain typically increases the survival, persistence and/or probiotic effect of a probiotic *L. reuteri* strain, manifested e.g. as an increase of mineral bio-accessibility, preferably an increase of mineral solubility and/or mineral absorption, in the intestinal tract of the patient; an increase in stress tolerance of a *L. reuteri* strain in the intestinal tract of the patient, an increase of stress tolerance to bile and gastric acid in the intestinal tract of the patient; an increase in adhesion of a *L. reuteri* strain to mucus in the intestinal tract of the patient; an increase of survival and/or engraftment and/or viability of a *L. reuteri* strain in the intestinal tract of the patient; an increase of epithelial integrity in the intestinal tract of the patient; an increase in bone formation and/or bone mineralization, and/or an increase in maturation and/or activity of osteoblasts of the patient. In these uses, the mineral is typically selected from magnesium, calcium and/or iron, preferably calcium and iron.

The inventive compositions may be also for use in the prevention and/or treatment of iron deficiency, preferably iron deficiency anemia, in a patient in need thereof. Alternatively, or additionally, the compositions may be also for use in the prevention and/or treatment of calcium and/or magnesium deficiency in a patient in need thereof. Moreover, the inventive compositions may be for use in the prevention and/or the treatment of bone loss and/or bone loss related disease in a patient in need thereof, and/or for use in promoting the bone formation and/or bone mineralization and/or bone strength in a patient in need thereof. Thereby, promoting the bone formation and/or bone mineralization and/or bone strength is promoting the maturation and/or activity of osteoblasts in a patient in need thereof. Moreover the inventive compositions may be for use in the prevention and/or treatment of a disease in a patient in need thereof, wherein the patient is suffering from a disrupted intestinal barrier, or disruptions in mucosal lining, preferably, wherein the disrupted intestinal barrier, or disruptions in mucosal lining is associated with a disease or condition selected from irritable bowel syndrome (IBS), Crohn's disease, ulcerative colitis, functional gastrointestinal disorders, such as functional abdominal pain, diarrhea, infantile colic, infectious diseases, depression, autism spectrum disorders, diverticular disease, periodontitis, osteopenia and/or osteoporosis. The inventive composition may be applied to a patient in need thereof, being a mammal, preferably selected form a child, a toddler or an infant, an elderly, a pregnant woman, a vegetarian, a lactating woman, a companion animal, a cat, or a dog, or being a bird, such as poultry. A patient in need thereof is typically a subject either being at risk of developing mineral deficiency or having developed a mineral deficiency or having any further disease or condition. The inventive compositions applied for such uses may be solid or liquid, and/or may be present in form of a powder, a tablet, a capsule, or may be in form of an oil formulation, an emulsion, an oil-in-water emulsion (o/w emulsion), or a water-in oil emulsion (w/o emulsion). The inventive composition for such uses may also be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage. As before, the *L. reuteri* strain is particularly preferable at least one *L. reuteri* strain or of multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

Further preferred embodiments are described herein below and in the dependent claims.

### BRIEF DESCRIPTION OF THE DRAWINGS

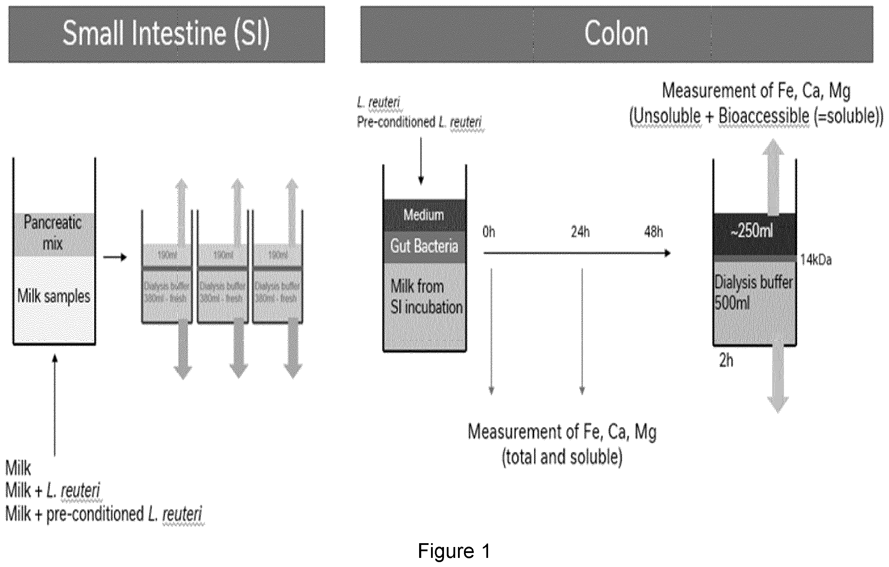

- **Figure 1:**: is an illustration of the short colonic SHIME model, which is a dynamic *in vitro* simulator model of the human digestion system and an experimental approach simulating the small intestine and colon incubations. The SHIME model allows the culturing of the complex intestinal microbial ecosystem under conditions representative for different intestinal regions.

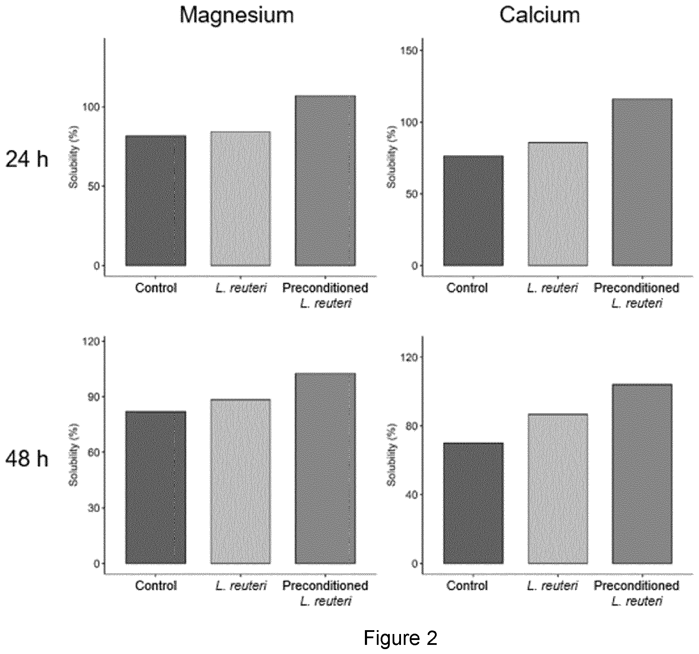

- **Figure 2:**: illustrates the solubility of magnesium and calcium at 24 h and 48 h in colon using the inventive pre-conditioned probiotic *L. reuteri* strain in a short colonic SHIME model. The results show that the pre-conditioned *L. reuteri* strain boosts magnesium and calcium solubility in colon. This suggests that the pre-conditioned *L. reuteri* strain can be used to promote magnesium and calcium absorption in a subject in need thereof.

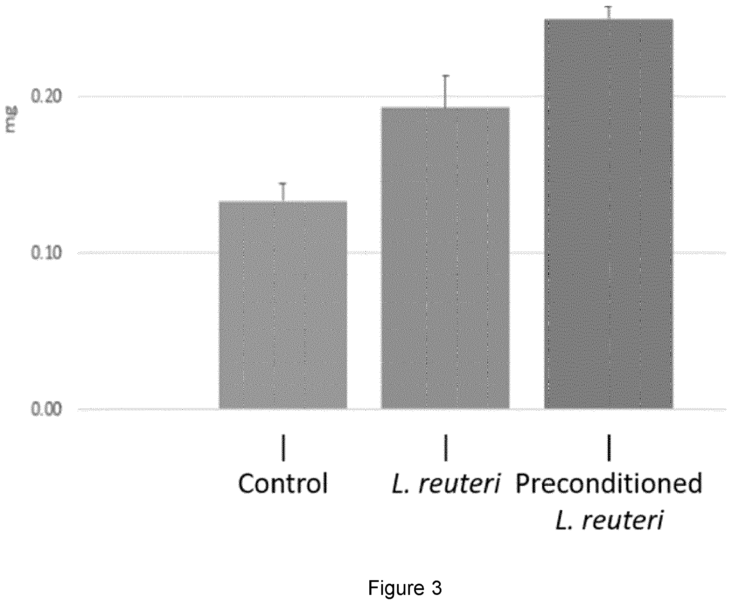

- **Figure 3:**: shows the iron bio-accessibility in the colon at 48 h using the inventive pre-conditioned probiotic *L. reuteri* strain in a short colonic SHIME model. The results show that the pre-conditioned *L. reuteri* strain boosts iron bio-accessibility in colon. This suggests that the pre-conditioned *L. reuteri* strain can be used to promote iron absorption in a subject in need thereof.

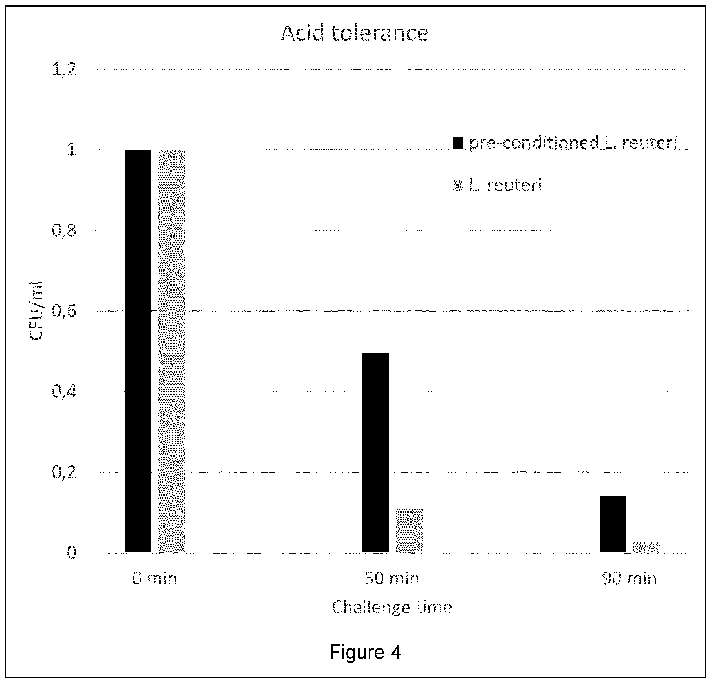

- **Figure 4:**: shows the acid stress tolerance for bacteria. The results show that the pre-conditioned *L. reuteri* strain is more tolerant to acid stress (normalized values). The results suggest that pre-conditioned *L. reuteri* strain will have higher survival and persistence in the GI tract.

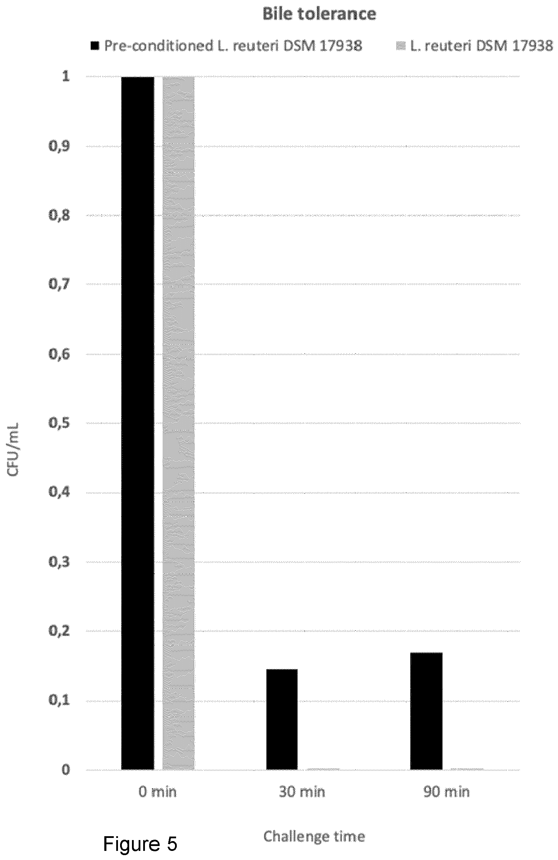

- **Figure 5:**: shows the bile stress tolerance for the pre-conditioned *L. reuteri* strain. The graph shows that the pre-conditioned *L. reuteri* strain has an increased resistance to bile (normalized values). The results suggest that pre-conditioned *L. reuteri* will have higher survival and persistence in the GI tract.

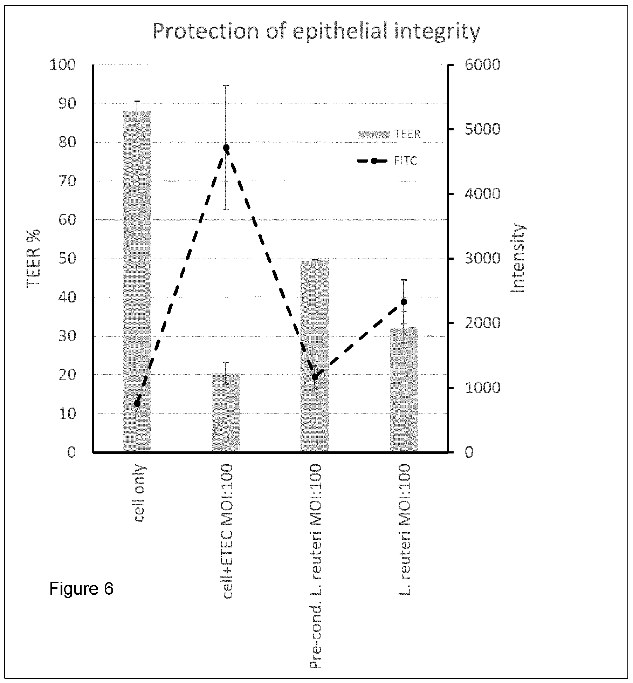

- **Figure 6:**: illustrates that the pre-conditioned *L. reuteri* strain gives an improved protection of epithelial integrity from detrimental effects of enterotoxigenic *Escherichia coli* (ETEC). The results suggest that pre-conditioned *L. reuteri* will have a protective effect of the epithelial barrier in the GI tract. Furthermore, the results also suggest that pre-conditioned *L. reuteri* improve mineral absorption in view of reduced damage of cells that are vital for this process.

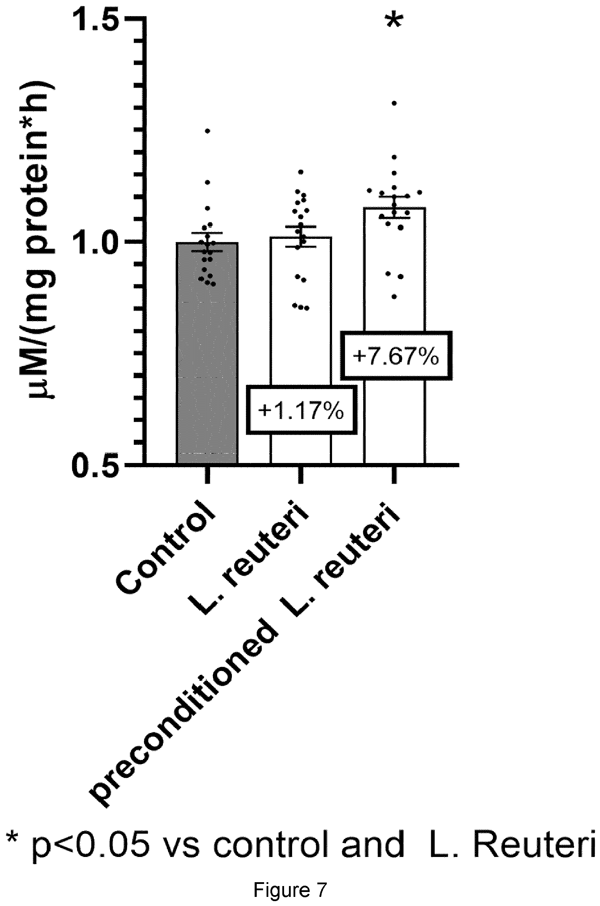

- **Figure 7:**: shows early differentiation of osteoblasts at Day 7, measuring alkaline phosphatase (ALP) activity. The results show that pre-conditioning of a *L. reuteri* strain led to increased ALP activity in osteoblasts, suggesting that the pre-conditioned *L. reuteri* strain can stimulate osteoblast differentiation.

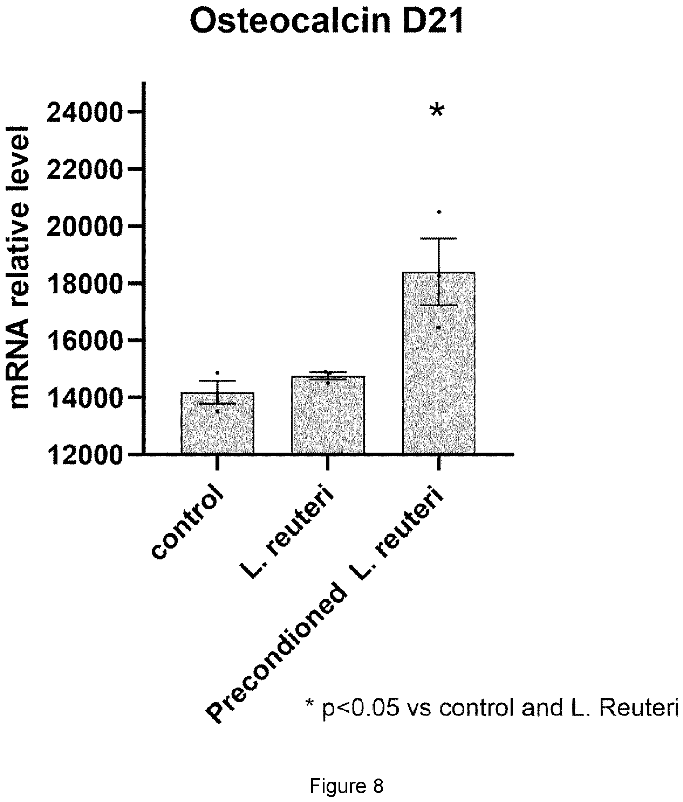

- **Figure 8:**: illustrates osteocalcin (oc) expression, a marker of mineralization that is expressed by differentiated osteoblasts. Results show that oc expression is enhanced by the pre-conditioned *L. reuteri* strain but not by a (not pre-conditioned) *L. reuteri* strain. This suggests that pre-conditioned *L. reuteri* strain can stimulate bone mineralization.

### DETAILED DESCRIPTION

The invention herein provides a way of enhancing survival, persistence and/or probiotic effects of *Lactobacillus reuteri* (*L. reuteri*) strains in a mammal or a bird, using specific substrate components during fermentation when manufacturing the probiotic *L. reuteri* strains. The inventors of the present invention have developed a method which comprises the use of galacto-oligosaccharides (GOS) during cultivation of the probiotic *L. reuteri* strain, so called pre-conditioning with GOS, which surprisingly results in an increased probiotic effect of the *L. reuteri* strain, particularly an increase of bioavailability of minerals, e.g., by increasing mineral solubility, accessibility and/or absorption of minerals, by improving the protective effect of the epithelial barrier integrity within the gastrointestinal tract and/or by improving bone formation and/or bone mineralization of a subject or patient upon administering the pre-conditioned probiotic *L. reuteri* strain, as described herein. This effect is surprisingly obtained even without the combined administration of GOS as a prebiotic and the pre-conditioned probiotic *L. reuteri* strain to a subject or patient in need thereof. Objectives of the invention therefore relate to a method for preparing such a pre-conditioned probiotic *L. reuteri* strain with GOS, to the non-therapeutic and therapeutic uses of a pre-conditioned probiotic *L. reuteri* strain to, among others, increase mineral solubility, accessibility and/or absorption in the gastrointestinal tract of a mammal or a bird.

Surprisingly, the pre-conditioned probiotic *L. reuteri* strains were also found to have an improved survival and/or persistence, particularly an improved mucus adhesion ability, improved stress tolerance to bile and gastric acid. These improved characteristics in turn may further also improve the probiotic effect of *L. reuteri* strains without the requirement of the presence of a prebiotic compound. Such improvements are particularly due to the use of GOS as a specific fermentation component for *L*. *reuteri* strains, termed herein pre-conditioning step. The improved survival, persistence and/or probiotic effect, such as induction of mineral (iron, magnesium and calcium) solubility of *L. reuteri* obtained by this additional step, as well as the improved survival, improved mucus adhesion ability, improved stress tolerance to bile and gastric acid, improved protective effect of the epithelial barrier integrity, improved bone formation and/or bone mineralization makes it possible to decrease the dosage and/or the frequency of administration or improves the effects to be achieved with the same dose of the probiotic required to obtain the sought after health effects.

The present invention therefore also aims to provide solutions, compositions and uses, which increase mineral solubility, bio-accessibility and hence also mineral absorption in the gastrointestinal tract. In detail, such solutions are compositions comprising galacto-oligosaccharides (GOS) pre-conditioned *L*. *reuteri* strain to be administered to a subject in need of increased mineral uptake. The increased mineral solubility, accessibility, and absorption takes place in the gastrointestinal tract, such as in the duodenum, the jejunum, the ileum and/or in the colon.

The present invention also aims to provide methods for pre-conditioning a probiotic *L. reuteri* strain such that increased mineral solubility and bioavailability and also mineral absorption in the gastrointestinal tract can be achieved by virtue of such a pre-conditioned *L. reuteri* strain to a composition.

In view of the above, the following is disclosed.

### METHODS FOR PRE-CONDITIONING A PROBIOTIC LACTOBACILLUS REUTERI BACTERIUM STRAIN

According to the first aspect the invention provides a method for preparing a pre-conditioned probiotic *Lactobacillus reuteri* strain characterized in that the method comprises the following steps:
a) cultivating or culturing a probiotic *L. reuteri* strain in the presence of GOS, thereby pre-conditioning the *L. reuteri* strain; and
b) harvesting the pre-conditioned probiotic *L. reuteri* strain from the growth medium.

In step a) of the inventive method, the probiotic *L. reuteri* strain is cultivated in the presence of GOS, thereby forming a pre-conditioned *L. reuteri* strain; In the context of the current invention, the term "pre-conditioning" of the probiotic *L. reuteri* strain preferably means growing, such as cultivating or fermenting, the probiotic *L. reuteri* strain with GOS during the process of producing the probiotic *L*. *reuteri* strain. Cultivation of a probiotic *L. reuteri* strain in the presence of GOS leads to formation of the pre-conditioned probiotic *L. reuteri* strain.

The inventive method therefore preferably comprises as a first step a) cultivating bacteria of a probiotic *L. reuteri* strain in the presence of GOS, thereby pre-conditioning the bacteria of the *L. reuteri* strain. In the inventive context, the terms "cultivation" and "fermentation" are used interchangeably.

In step b) the pre-conditioned bacteria of the probiotic *L. reuteri* strain are then harvested from the growth medium. The term "pre-conditioned probiotic *L. reuteri strain"* refers to a probiotic *L. reuteri* strain produced, obtained or obtainable by the inventive method including a pre-conditioning step with GOS. Correspondingly, the term "pre-conditioned bacteria of a probiotic *L. reuteri* strain" refers to bacteria of a probiotic *L. reuteri* strain produced, obtained or obtainable by the inventive method including a pre-conditioning step with GOS.

A *Lactobacillus reuteri* strain as used throughout the current invention may generally be any *L. reuteri* strain, more preferably any commercially available *L. reuteri* strain. In this context, the terms *"Lactobacillus reuteri* strain" and *"Lactobacillus reuteri* bacteria" may be used synonymously. It is noted thereby that in the last decades DNA analysis tools have become more sophisticated, which has enabled scientists to discover many new bacterial species as well as realizing that the species historically grouped under the *Lactobacillus* genus were too diverse and did not conform to nomenclature conventions. To keep the probiotic groups accurate and organized, the genus *Lactobacillus* was therefore split into 25 different genera, including 23 novel genera. As a result, many probiotics have recently been given new genus names. Therefore, an alternative genus name for *Lactobacillus reuteri* is *Limosilactobacillus reuteri.* Since the reclassification process was very recent, we will use the former genera nomenclature, i.e., *Lactobacillus* for clarity purposes in this application. The revised genera nomenclature and alternative names should however be readily obtainable by anyone using available reclassification tools.

Currently preferred bacteria provided in the compositions are bacteria of at least one *L. reuteri* strain or of multiple *L. reuteri* strains, preferably as mentioned herein.

In a preferred embodiment of the current invention, a *L. reuteri* strain as used herein is *L. reuteri* strain DSM 17938. *Lactobacillus reuteri* DSM 17938 was deposited under the Budapest Treaty at the DSMZ-Deutsche Sammlung von Mikroorganismen und Zellkulturen GmbH (Mascheroder Weg 1b, D-38124 Braunschweig, Germany) on January 30, 2006.

Alternatively or additionally, the *L. reuteri* strain as used herein may be provided from at least one *L*. *reuteri* strain selected from *L. reuteri* as deposited under ATCC PTA 5289, *L. reuteri* strain ATCC PTA 6475, *L. reuteri* DSM 32846, *L. reuteri* DSM 32848, *L. reuteri* DSM 32849, *L. reuteri* DSM 27131, *L. reuteri* DSM 32465 or *L. reuteri* strain ATCC PTA 4659.

*Lactobacillus reuteri* DSM 27131 was deposited under the Budapest Treaty at the Leibniz Institut DSMZ-Deutsche Sammlung von Mikroorganismen und Zellkulturen GmbH (Inhoffenstr. 7B, D-38124 Braunschweig, Germany) on April 18, 2013.

*Lactobacillus reuteri* DSM 32846, DSM 32848 and DSM 32849 were deposited under the Budapest Treaty at the Leibniz Institut DSMZ-Deutsche Sammlung von Mikroorganismen und Zellkulturen GmbH (Inhoffenstr. 7B, D-38124 Braunschweig, Germany) on July 4, 2018.

*Lactobacillus reuteri* DSM 32465 was deposited under the Budapest Treaty at the Leibniz Institute DSMZ-German collection of Microorganisms and Cell Cultures (Inhoffenstr. 7B, D-38124 Braunschweig, Germany) on March 21, 2017.

*Lactobacillus reuteri* ATCC PTA 5289 was deposited under the Budapest Treaty at the American Type Culture Collection (10801 University Blvd., Manassas, VA 20110-2209, U.S.) on June 25, 2003.

*Lactobacillus reuteri* ATCC PTA 6475 was deposited under the Budapest Treaty at the American Type Culture Collection (10801 University Blvd., Manassas, VA 20110-2209, U.S.) on December 21, 2004.

*Lactobacillus reuteri* ATCC PTA 4659 was deposited under the Budapest Treaty at the American Type Culture Collection (10801 University Blvd., Manassas, VA 20110-2209, U.S.) on September 11, 2002.

The term "GOS" as used herein means "Galacto-oligosaccharide". Galacto-oligosaccharides (GOS) as used herein typically consist of β-linked galactose moieties with galactose or glucose at the reducing end. Such GOS contains β-(1→2), β-(1→3), β-(1→4), or β-(1→6) linked galactose moieties and may have a degree of polymerization (DP) of 3-8 galactose units. The term GOS is therefore preferably referred to as oligosaccharide(s) comprising at least three galactose units, more preferably as oligosaccharide(s) comprising at least four galactose units, preferably having a degree of polymerization (DP) of 3-8 galactose units. The GOS added to the growth medium in step a) therefore preferably has a degree of polymerization (DP) of 3-8.

Oligosaccharides occur naturally in the milk of some mammals, e.g., cow and human. GOS are commercially available and can be synthesized via biosynthesis processes from lactose by trans-galactosidase activity of β-galactosidases. GOS formation in biosynthesis procedures is usually favored by high concentrations of lactose or lactulose, incomplete lactose turnover, low water activity, and the use of enzymes with preference for trans-galactosylation. The linkage type(s) of resulting GOS from such processes is(are) specific for the enzymes used for biosynthesis.

One particular type of mixture of carbohydrates that can advantageously be used herein as a source of GOS to grow the probiotic bacteria is a mixture of GOS and of cow's milk oligosaccharides. In particular mixtures of GOS with 3'-sialyllactose (3'-SL) and/or 6'-sialyllactose (6'-SL) are preferably used. Indeed, such mixtures contain some oligosaccharides similar to human milk, which is particularly advantageous when the composition of the invention is used as an infant formula or as a nutritional supplement for infants. Such advantageous effects are described for example in Simeoni et al.; "Gut microbiota analysis reveals a marked shift to bifidobacteria by a starter infant formula containing a synbiotic of bovine milk-derived oligosaccharides and Bifidobacterium animalis subsp. lactis CNCM I-3446"; Environ Microbiol, 2016, 18(7): 2185-2195. Such compositions can typically be obtained from concentrating whey permeate to obtain a concentrated bovine milk oligosaccharide composition and either adding GOS or generating the GOS in situ from hydrolysis of lactose by the action of a β-galactosidase.

The GOS source used in step a) of the inventive method to pre-condition the probiotic *L. reuteri* strain can be provided in the form of essentially pure GOS (i.e., ingredient having at least 90% GOS) or as part of a mixture, such as a mixture of carbohydrates, comprising GOS. The essential aspect is that a sufficient amount of GOS is provided in the growth medium. According to one embodiment the GOS source is therefore typically added to the growth medium in step a) in an amount of at least 0.2 wt%, preferably at least 0.5 wt%, even more preferably at least 0.75 wt% of GOS in the growth medium, and preferably at most 8 wt% of GOS, such as at most 7 wt% GOS, at most 6 wt% GOS, or even at most 5 wt% GOS, more preferably at most 4 wt% of GOS or even at most 3% GOS, at most 2 wt% of GOS, or at most 1 wt% of GOS in the growth medium, preferably calculated on basis of the growth medium (% w/w). Any combination of these upper and lower ranges is encompassed herewith. More preferably, GOS is added to the growth medium in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium (% w/w). Addition of GOS may occur preferably at the start, in the middle or at the end of cultivation of a probiotic *L. reuteri* strain in step a), and/or at once, stepwise or continuously.

When a mixture of carbohydrates is used as a source of GOS, it is preferred that the amount of GOS in such a mixture is at least 20 wt%, preferably at least 30 wt%, more preferably at least 40 wt%, even more preferably at least 45 wt%, most preferably at least 48 wt%, preferably calculated on a dry weight basis of the mixture containing GOS. Although it is not necessary that the bacteria consume only GOS as carbon source during the fermentation, high proportion of GOS in the GOS source, favors the consumption of GOS by the bacteria during fermentation over consumption of other carbohydrates, leading to improved pre-conditioning effect. Smaller carbohydrates such as di-saccharides (lactose for instance) can also be present and be consumed by the bacteria supporting the pre-conditioning effect. Accordingly, it is also preferred that the GOS source comprises at most 55 wt%, preferably at most 50 wt%, more preferably at most 45 wt%, most preferably at most 42 wt% of mono- or di-saccharides and/or no more than 40 wt%, preferably no more than 30 wt% lactose, preferably calculated on a dry weight basis of the mixture containing GOS. As an example, a mixture of carbohydrate used as a source of GOS may comprise GOS in amounts defined above for the mixture and the remainder formed by lactose, glucose, and/or galactose, and optionally 3'-SL and/or 6'-SL.

In an embodiment, the culture medium also comprises an electron acceptor, such as fructose, citrate, glycerol and/or 1,2-propanediol.

In a particular embodiment, the culture medium also comprises fructose, such as acting as an electron acceptor. In illustrative, but non-limiting, examples the culture medium may comprise at least 0.2 wt% fructose, preferably at least 0.5 wt% fructose, more preferably at least 1 wt% fructose, even more preferably at least 1.5 wt% fructose calculated on a dry weight basis of the culture medium. Correspondingly, in illustrative, but non-limiting, examples the culture medium preferably does not comprise more than 16 wt% fructose, preferably no more than 14 wt% fructose, more preferably no more than 12 wt% fructose and even more preferably no more than 10 wt% fructose. Any combination of these upper and lower ranges is encompassed herewith. In an embodiment, the culture medium comprises a wt% ratio between the electron acceptor (e.g. fructose) and GOS selected within a range of from 0.25:1 to 5:1, preferably within a range of from 0.5:1 to 4:1, and more preferably within a range of from 0.75 to 2.5:1. Particularly, preferred wt% ratios between the electron acceptor (e.g. fructose) and GOS are within a range of from about 1:1 to about 2:1.

According to a further embodiment, addition of GOS in step a) of the method for preparing the pre-conditioned probiotic *L. reuteri* strain to the growth medium may occur at any point of time of the cultivation step, e.g., at the start of cultivation of the probiotic *L. reuteri* strain, at once, stepwise or continuously during the cultivation step a) of the probiotic *L. reuteri* strain, more preferably at the start of cultivation of the probiotic *L. reuteri* strain. Alternatively, or even additionally, addition of GOS in step a) of the method for preparing the pre-conditioned probiotic *L. reuteri* strain to the growth medium may also be in the middle or at the end of cultivation step a).

The cultivation step a) is carried out in a way that is well known to the person skilled in the art. Cultivation in step a) includes the steps of inoculation of sterile standard growth medium with a defined amount of bacteria (cfu (colony forming units)), followed by incubation under defined temperature (usually 37°C) and pH. In this context, a defined amount of bacteria (cfu) for starting an inoculation of sterile standard growth medium may be determined by a skilled person according to common general knowledge and practice in the art. Suitable yields can be obtained with the growth medium comprising GOS as described above, preferably without changing the cultivation conditions compared to what the person skilled in the art would use for cultivation of the same strain with a standard growth medium.

A standard growth medium for cultivating/fermenting the *L. reuteri* strain in step a) of the current invention may be any known standard growth medium used for lactobacilli (LAB), such as MRS medium, or any further suitable medium. Such a standard growth medium is preferably commercially available but may be also produced by addition of compounds according to well-known standard recipes. A standard growth medium for *L. reuteri* strains may typically contain carbohydrates, such as simple sugars selected e.g., from dextrose, sucrose, maltose, fructose or lactose, various nitrogen sources, such as peptone, yeast extract, beef extract, or whey protein, minerals, mainly Mn²⁺ and Mg²⁺, and buffering agents, such as sodium acetate (CH₃COONa), trisodium citrate (Na₃C₆H₅O₇), or Disodium-glycerophosphate (C₃H₇Na₂O₆P) are commonly used buffers in LAB media. Other components that have been used in standard growth media with buffering activity may include disodium phosphate (Na₂HPO₄), ammonium citrate (NH₄C₆H₅O₇), trisodium phosphate (Na₃PO₄), potassium biphosphate (KH₂PO₄), magnesium phosphate tribasic Mg₃(PO₄)₂, calcium carbonate (CaCO₃), and dipotassium phosphate (K₂HPO₄). Such standard growth media may also contain a wide range of growth factors, surfactants, such as lecithin or Tweens (e.g., Tween 20, 80 and 85). The cultivation/fermentation may be carried out under anaerobic or aerobic conditions, depending on the strain to be produced, but preferably under anaerobic conditions. Also, the pH may be controlled or not, depending on the conditions known to be the best for a specific strain to grow. The temperature and duration of the cultivation step is variable from one strain to another and is also well-known to the person skilled in the art of probiotic *L. reuteri* strain cultivation.

According to one embodiment the cultivation/fermentation of the probiotic *L. reuteri* strain in step a) of the inventive method occurs over a considerable period of time to allow a pre-conditioning of the probiotic *L. reuteri* strain in the presence of GOS, typically for a period of at least 1 hour, preferably for a period of between 5 to 18 hours, likewise preferably 7 to 14 hours, more preferably for a period of between 10 to 13 hours, and even more preferably for a period of about 12 hours.

Moreover, according to a further embodiment the cultivation/fermentation of the probiotic *L. reuteri* strain in step a) of the inventive method occurs at a temperature of between 25 to 45°C, preferably between 30 and 40°C, and even more preferably at a temperature of between 35 and 39°C, such as about 37°C.

In another embodiment the cultivation/fermentation of the probiotic *L. reuteri* strain in step a) of the inventive method occurs at a pH of between 3.0 and 7. The pH during pre-conditioning of probiotic *L*. *reuteri* strain during cultivation step a) may be either not controlled or set to a constant value throughout the cultivation step a), typically to be monitored and adjusted during cultivation step a). Accordingly, the pH may be between 3.0 and 7, particularly when pH is not controlled during pre-conditioning of probiotic *L. reuteri* strain. Alternatively, if pH is controlled during cultivation step a), pH may be adjusted to a range of about 5.0 to 7.0, e.g., about 5.0 to 6.0, about 5.5 to 6.5 (e.g., about 6.0±0.2) or about 6.0 to 7.0 (e.g., about 6.6±0.2), more preferably pH may be adjusted to a range of about 5.5 to 6.5, even more preferably pH may be adjusted to a range of about 6.0±0.4, and most preferably pH may be adjusted to a range of about 6.0±0.2.

After cultivation/fermentation of a probiotic *L. reuteri* strain in step a) of the inventive method the thereby pre-conditioned probiotic *L. reuteri* strains are harvested in step b). The harvesting step, which aims at separating the bacterial cells from the growth medium, is also carried out in a way that is well known to the person skilled in the art, such as by concentrating the bacteria, centrifugation, filtration, membrane-filtration, decantation, isolation, purification, etc., preferably centrifuging or concentrating the probiotic *L. reuteri* strain. Typically, harvesting of the pre-conditioned probiotic *L. reuteri* strain occurs in step b) after a period of at least 1 hour as defined above for cultivation/fermentation in step a). The harvesting in step b) preferably leads to an isolated or, at least partly, purified pre-conditioned probiotic *L. reuteri* strain.

After harvesting the pre-conditioned probiotic *L. reuteri* strain in step b) the harvested cells may be washed in an optional step c) prior to further processing, typically to remove traces of growth medium (after cultivation/fermentation). Washing may occur with either saline water, brine, or any further suitable liquid.

Either directly after harvesting the pre-conditioned probiotic *L. reuteri* strain from the growth medium according to step b) or after an optional washing step c) the pre-conditioned probiotic *L. reuteri* strain may be subjected to a drying step d). Such a drying step d) may be carried out by any method known to a skilled person, such as spray-drying, fluid bed drying, air convective drying, atmospheric drying, roller drying or freeze drying, lyophilizing, and more preferably spray-drying or freeze drying. Spray drying may also be carried out using in the presence of protecting agents, e.g., as described in WO 2017/001590.

Optionally, the pre-conditioned probiotic *L. reuteri* strain obtained after harvesting from the growth medium and/or after washing the pre-conditioned probiotic *L. reuteri* strain may further be mixed with protective agents, such as cryoprotectants, lyoprotectants and/or carriers before the drying step, as known to the person skilled in the art and as appropriate depending on the drying method to be used and the bacteria to be dried. The drying step d) is therefore preferably preceded by treating the harvested pre-conditioned probiotic *L. reuteri* strain with a protectant, a cryoprotectant, a lyoprotectant and/or a carrier. Such protectants and/or carriers typically include glycerol, skimmed milk, serum albumin, peptone, yeast extract, saccharose, glucose, sorbitol, malt extract, trehalose etc. Commercially available cryoprotectants include e.g., cryoprotectant 'Unipectine^{™} RS 150, etc. or are as described in EP 3016511, US 2014/0004083 A1, US 2016/0298077 A1, etc.

Alternatively, or additionally, the pre-conditioned probiotic *L. reuteri* strain obtained after harvesting from the growth medium or after washing or even after drying the pre-conditioned probiotic *L. reuteri* strain, i.e., obtained after any of steps b), c) or d) may be encapsulated for further preservation and/or use, e.g. by formation of microspheres containing the pre-conditioned probiotic *L. reuteri* strain using common encapsulation agents, e.g. alginate, alginate/pullulan, starch, xanthan, xanthan-gellan gum mixtures, gelatin, cellulose acetate phethalate, chitosan, or any further suitable encapsulation material. Encapsulation is well known and can be carried out by a person skilled in the art.

### COMPOSITIONS COMPRISING THE PRE-CONDITIONED PROBIOTIC L. REUTERI STRAIN

According to the second aspect, the invention provides a composition comprising an effective amount of a pre-conditioned probiotic *L. reuteri* strain, wherein the pre-conditioned probiotic *L. reuteri* strain has been prepared/obtained by cultivating a probiotic *L. reuteri* strain in the presence of GOS. Such a pre-conditioning of a probiotic *L. reuteri* strain in the presence of GOS is typically carried out according to the inventive method as described herein under the first aspect of the invention for preparing a pre-conditioned *L. reuteri* strain and also as defined in the claims as attached. As already disclosed above in detail, cultivating/fermentation of a probiotic *L. reuteri* strain in the presence of GOS preferably occurs over a time sufficient to pre-condition a probiotic *L. reuteri* strain accordingly, e.g., over a period of at least one hour, preferably according to a process as defined herein.

A related aspect of the invention defines a pre-conditioned probiotic *L. reuteri* strain obtained or obtainable by cultivating a probiotic *L. reuteri* strain in the presence of GOS.

As before, the *L. reuteri* strain is particularly preferably at least one *L. reuteri* strain or multiple *L. reuteri* strains as defined above, preferably selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, more preferably the probiotic *L. reuteri* strain is *L. reuteri* strain as deposited under DSM 17938.

In one embodiment, the *L. reuteri* strain is particularly preferably at least one *L. reuteri* strain or multiple *L. reuteri* strains as defined above, preferably selected from group comprising or consisting *of L. reuteri* strain as deposited under DSM 17938, *L. reuteri* strain as deposited under ATCC PTA 5289, *L. reuteri* strain as deposited under ATCC PTA 6475, and *L. reuteri* strain as deposited under ATCC PTA 4659.

In one embodiment, the *L. reuteri* strain is particularly preferably at least one *L. reuteri* strain or multiple *L. reuteri* strains as defined above, preferably selected from group comprising or consisting *of L. reuteri* as deposited under DSM 32846, *L. reuteri* as deposited under DSM 32848, *L. reuteri* as deposited under DSM 32849, *L. reuteri* as deposited under DSM 27131, and *L. reuteri* as deposited under DSM 32465.

Typically, an "effective amount" of a pre-conditioned probiotic *L. reuteri* strain as defined herein may comprise a pre-conditioned probiotic *L. reuteri* strain typically in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose, or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose, more preferably around 10⁷ cfu to 10⁹ cfu per daily dose. A preferred daily dose is around 10⁸ total cfu per daily dose, 10⁷ to 10⁹ cfu per daily dose or 10⁸ to 10⁹ cfu per daily dose.

The pre-conditioning of the probiotic *L. reuteri* strain improves or increases the survival, persistence and/or probiotic effect of the *L. reuteri* strain. Such an improvement or increase of the survival, persistence and/or probiotic effect may be e.g.
- an increase of mineral bioavailability, preferably an increase of mineral solubility and/or mineral absorption, in the intestinal tract of a subject; and/or
- an increase in stress tolerance of *L. reuteri* strain in the intestinal tract of a subject, preferably an increase of stress tolerance to bile and gastric acid in the intestinal tract of a subject; and/or
- an increase in adhesion of *L. reuteri* strain to mucus in the intestinal tract of a subject; and/or
- an increase of survival and/or engraftment and/or viability of a *L. reuteri* strain in the intestinal tract of a subject; and/or
- an increase of epithelial integrity in the intestinal tract of a subject; and/or
- a promotion of bone formation and/or bone mineralization of a subject; and/or
- a promotion of the maturation and/or activity of osteoblasts of a subject.

Accordingly, the pre-conditioning of the probiotic *L. reuteri* strain modifies the probiotic *L. reuteri* strain so that the *L. reuteri* strain have different and improved properties as compared to a non-pre-conditioned probiotic *L. reuteri* strain, i.e., a probiotic *L. reuteri* strain that does not have been pre-conditioned by cultivating or culturing in the presence of GOS. These different and improved properties include, among others, the above listed and in Examples 5 to 10 disclosed properties.

The inventive composition comprising an effective amount of a pre-conditioned probiotic *L. reuteri* strain, as defined herein may be any type of composition, in which probiotic bacteria can be incorporated, such as a food product, a beverage, an animal feed product, a nutritional supplement for human or animal, a pharmaceutical composition or a cosmetic composition. More preferably, the inventive composition may be provided in an administrable form, preferably selected from the group consisting of food products, beverages, pharmaceutical formulations, dietary or nutritional supplements, functional food products, functional beverage products, nutraceuticals, and combinations thereof.

The inventive composition may be solid or liquid. Preferably it is present in form of a powder, a tablet, a capsule, or may be in form of an oil formulation, an emulsion, e.g., an oil-in-water emulsion (o/w emulsion), a water-in oil emulsion (w/o emulsion), etc. If present in powder form it can be intended to be used by the final consumer in solid (such as powder form) or semi-solid form (such as for example in the form of a paste) or, alternatively, to be reconstituted into a liquid before use.

Food products and beverages as defined herein may include all products intended to be consumed orally by human beings, for the purpose of providing nutrition and/or pleasure. The expression "food product" as well as the term "beverages" usually mean compositions which nourish a subject. This "food product" is usually to be taken orally or intraperitoneally, and it can include a lipid or fat source and a protein source. Likewise, a "beverage" is usually to be taken orally, is liquid or a semi-liquid, and can include a lipid or fat source and a protein source.

Food products and beverages as defined herein can, for example, include a nutritional composition, preferably for human consumption, such as for infants and/or young children, for a pregnant or lactating woman or a woman desiring to get pregnant, for individuals in need of a special nutrition due to an adverse health condition or for elderly people. More preferably, the nutritional composition is selected from infant formula, infant cereals, follow-up or follow-on formula, growing-up milks, functional milks, baby food, infant cereal compositions, and milk products for pregnant and lactating women or for women desiring to get pregnant. Other examples of food products and beverages include sweet and savory snacks, powdered drinks, cereal products and dairy products, such as milk products, whey protein-based products, etc. According to one particular embodiment, the inventive composition is an infant formula, a follow-on formula, a growing-up milk or a product for pregnant or lactating women. In one further particular embodiment the inventive composition may be an infant formula.

The inventive composition can also be in the form of an animal food product or a nutritional supplement for animals (such as mammals or birds). Preferably, the animal is a mammal. Examples of animals include, cows, sheep, goats, horses, dogs, cats, rabbits, rats, mice, fish, birds (such as poultry) and the like.

The expression "infant formula" as used herein refers to a foodstuff intended for particular nutritional use by infants during the first months of life and satisfying by itself the nutritional requirements of this category of person (reference: Article 2(c) of the European Commission Directive 91/321/EEC 2006/141/EC of 22 December 2006 on infant formulae and follow-on formulae; Reg 609/2013 art 2.1.c and Reg 2016/127). It also refers to a nutritional composition intended for infants and as defined in Codex Alimentarius (Codex STAN 72-1981) and Infant Specialities (incl. Food for Special Medical Purpose). The expression "infant formula" encompasses both "starter infant formula" and "follow-up formula" or "follow-on formula", and includes compositions to be provided to premature infants.

A "follow-up formula" or "follow-on formula" is an infant nutritional composition given from the 6^{th} month onwards. It constitutes the principal liquid element in the progressively diversified diet of this category of person.

The expression "baby food" means a foodstuff intended for particular nutritional use by infants or young children during the first years of life.

In the inventive context, the term "infant" means a child under the age of 12 months. Moreover, the expression "young child" means a child aged between one and three years, also called toddler.

The expression "infant cereal composition" means a foodstuff intended for particular nutritional use by infants or young children during the first years of life.

The product can also be in the form of an animal food product or a nutritional supplement for animals. Preferably, the animal is a mammal or a bird. Examples of animals include, cows, sheep, goats, horses, dogs, cats, rabbits, rats, mice, fish, birds (such as poultry) and the like.

Dietary or nutritional supplements are typically present in the form of a liquid, such as a refrigerated liquid, in form of a powder or a tablet or capsule, an oil formulation, an emulsion, e.g., an oil-in-water emulsion (o/w emulsion), a water-in oil emulsion (w/o emulsion), etc. as mentioned above. Preferably it is in the form of a powder, a tablet, a capsule or an oil formulation. Powder supplements typically encompass supplements to be dissolved in a liquid or to be sprinkled on food or in a beverage. Such supplements are intended to provide additional nutrients and/or a health benefit to the subject consuming it, as well as other beneficial ingredients, such as the herein-defined and prepared pre-conditioned probiotic *L. reuteri* strain. A supplement according to the present invention can therefore be used for providing nutrients and/or a health benefit to human beings, as well as to animals, as defined above. Dietary or nutritional supplements include for example the herein-defined and prepared pre-conditioned probiotic *L. reuteri* strain as a powder supplement to be added to any sort of dietary or nutritional composition.

Pharmaceutical products include powder, tablet or capsule products intended to treat or prevent an adverse medical condition in a subject in need thereof, or to promote a favorable health condition.

Cosmetic compositions are typically intended for an aesthetic effect on the body and may be for topical use or may be administered by oral route, in the form of a powder, tablet or capsule.

Optionally, the inventive composition may comprise additionally to the herein-defined and prepared pre-conditioned probiotic *L. reuteri* strain also GOS to further provide improved properties to the final product. Such improved properties may be synergistic effects of the combined administration of the pre-conditioned probiotic *L. reuteri* strain and galacto-oligosaccharides. If GOS are contained, such GOS are preferably as defined above. Moreover, such GOS may be comprised in the inventive composition in an amount of at least 1 or 2 g per daily dose, preferably at least 3 g per daily dose, likewise preferably between 2 to 12 g per daily dose, or between 2 to 10 g per daily dose, between 2 to 8 g per daily dose, or between 2 to 7 g per daily dose, more preferably between 3 to 12 g per daily dose, likewise more preferably between 3 and 10 g per daily dose, such as between 3 to 8 g per daily dose, between 3 to 7 g per daily dose, between 3 to 6 g per daily dose, or about 2, 3, 4, 5, 6, 7, 8, 9, 10, 11, or 12 g per daily dose or any range formed thereby.

According to a particular embodiment, no GOS is added to or contained in the inventive composition. In this particular embodiment, GOS is preferably used only to pre-condition the *L. reuteri* strain in step a) of the inventive method and not further added when preparing the inventive composition. Hence, in a particular embodiment, the inventive composition lacks any GOS, optionally with exception to trace amounts of GOS remaining following harvesting the pre-conditioned probiotic *L. reuteri* strain from the culture medium comprising GOS. Trace amount as used herein means less than 5 mg GOS per g of the composition, preferably less than 1 mg GOS per g of the composition, more preferably less than 0.5 mg GOS per g of the composition, and even more preferably less than 0.1 mg GOS per g, and most preferably less than 0.01 mg GOS per g of the composition.

### APPLICATIONS OF INVENTIVE COMPOSITIONS COMPRISING THE PRE-CONDITIONED PROBIOTIC L. REUTERI STRAIN

The present invention aims to provide compositions and uses, which improve or increase the probiotic effect and/or increase mineral solubility and bioavailability and also mineral absorption in the gastrointestinal tract.

As shown herein, inventive compositions allow for enhancing probiotic effects of *L. reuteri* strains in a mammal or a bird, using GOS as specific substrate components during fermentation when manufacturing the probiotic *L. reuteri* strains. The herein disclosed method specifically pre-conditions the probiotic *L. reuteri* strain by use of GOS during cultivation of the probiotic *L. reuteri* strain, such that an increased probiotic effect of the *L. reuteri* strain occurs *in vivo,* particularly during non-therapeutic and therapeutic administrations as disclosed herein. This particularly enables and allows an increase of bioavailability of minerals, e.g., by increasing mineral solubility, accessibility and/or absorption of minerals within the gastrointestinal tract of a subject or patient upon administering the pre-conditioned probiotic *L. reuteri* strain or the composition, as described herein. This surprising effect can be obtained even without the combined administration of GOS as a prebiotic and the pre-conditioned probiotic *L*. *reuteri* strain to a subject or patient in need thereof.

Surprisingly, as already said initially, the pre-conditioned *L. reuteri* strains were also found to have an increased survival and persistence, particularly an improved mucus adhesion ability, improved stress tolerance to bile and gastric acid. These improved characteristics, in turn, may further also improve the probiotic effect of *Lactobacillus reuteri* strains without the requirement of the presence of a prebiotic compound. The improved survival, persistence and/or probiotic effect, exemplified as the induction of mineral (iron and calcium) solubility of the *Lactobacillus reuteri* strain obtained by the pre-conditioning step as well as the improved mucus adhesion ability, improved stress tolerance to bile and gastric acid, improved protective effect of the epithelial barrier integrity, improved bone formation and/or bone mineralization conferred to a subject and/or patient in need thereof by administering the inventive composition comprising the pre-conditioned probiotic *L. reuteri* strain makes it possible to decrease the dosage and/or the frequency of administration of the probiotic required or to achieve better effects with the same dose of the probiotic to obtain the sought after health effects or therapeutic effects.

Moreover, inventive compositions are compositions comprising GOS pre-conditioned probiotic *L. reuteri* strain to be administered to a subject or a patient in need thereof, e.g., a subject or a patient in need of increased mineral uptake. The increased mineral solubility, accessibility, and absorption takes place in the gastrointestinal tract, such as in the duodenum (first part of small intestine), the jejunum (middle part of small intestine), the ileum (final part of small intestine) and/or in the colon (large intestine). The present invention also aims to provide methods for pre-conditioning a probiotic *L. reuteri* strain such that increased mineral solubility and bioavailability and also increased mineral absorption in the gastrointestinal tract can be achieved by virtue of such pre-conditioned probiotic *L. reuteri* strains to a composition.

As already outlined above, minerals are essential for all living species, even though the specific requirements differ between species. Mineral deficiencies can lead to disease and so can mineral excess. Correct amounts of minerals at the correct ratios are thus required for optimal health. Most natural diets will provide minerals in appropriate balances, but there are situations when a balanced diet is not enough to maintain health with respect to minerals. As mentioned, minerals are essential and have a great number of important functions in an organism.

Iron, for example, is responsible for the transport of oxygen and myoglobin. Moreover, iron is part of many enzymes such as catalases, peroxidases and cytochromes. Iron is generally absorbed in the duodenum and the upper part of the small intestine in the form of Fe²⁺ by cells that line the gastrointestinal tract, which absorbs iron from the ingested food.

Calcium is another essential mineral which is inter alia an important bone component and an enzyme activator. Calcium also takes part in conduction of bioelectric impulses, blood coagulation, muscle contraction, inflammation and hormonal secretion. Calcium is generally absorbed in the small intestine in the presence of the active form of vitamin D (calcitriol).

Magnesium is a bone component and takes part in neuronal conduction and ribosomal processes, among many other functions. It is also generally absorbed in the small intestine.

A mineral deficiency of any of such minerals can lead to severe complications, conditions and diseases. The following uses, both non-therapeutic and therapeutic, are intended to address such mineral deficiency either by preventing or treating mineral deficiencies, e.g., in healthy patients in a non-therapeutic manner, e.g., by providing the inventive compositions as nutritional supplements or beverage, or in a patient in need thereof by administering or supplying the composition as defined above, e.g., as nutritional composition or beverage, as a pharmaceutical composition, etc.

The present invention also provides methods, compositions and uses which increase mineral solubility and bioavailability and also mineral absorption in the gastrointestinal tract using the GOS pre-conditioned probiotic *L. reuteri* strain. The present invention furthermore comprises methods, compositions and uses to improve acid and bile tolerance of the probiotic *L. reuteri* strain in the gastrointestinal tract, particularly by administering or using the GOS pre-conditioned probiotic *L. reuteri* strain, thereby increasing survival and persistence of the pre-conditioned probiotic *L. reuteri* strain in the GI tract.

Moreover, alternatively, or additionally, the inventive compositions are compositions comprising galacto-oligosaccharides (GOS) pre-conditioned probiotic *L. reuteri* strain to be administered to a subject or a patient in need thereof, e.g., a subject or a patient in need of increased bone strength or a subject or a patient at risk of developing osteoporosis. Moreover, the inventive compositions are to be administered to a subject or a patient in need thereof, wherein the subject or patient is at risk for or is suffering from a disrupted intestinal barrier, or disruptions in mucosal lining.

In the following, without being limiting thereto, exemplary therapeutic and non-therapeutic applications (uses) are listed. These therapeutic and non-therapeutic applications apply not only to the pre-conditioned probiotic *L. reuteri* strain but also to compositions of the invention.

### • NON-THERAPEUTIC USES OF COMPOSITIONS COMPRISING THE PRE-CONDITIONED PROBIOTIC L. REUTERI STRAIN

According to the third aspect, the invention provides a non-therapeutic use of an effective amount of a pre-conditioned probiotic *L. reuteri* strain as prepared according to the invention or as described herein, typically in form of an administrable composition, to increase or boost the survival, persistence and/or probiotic effect of the *L. reuteri* strain in the digestive tract of a healthy subject. Such an increase or boosting of the survival, persistence and/or probiotic effect by the pre-conditioned probiotic *L. reuteri* strain in the digestive tract of a healthy subject is typically to be determined in comparison with the not yet pre-conditioned probiotic *L. reuteri* strain in the digestive tract of a healthy subject.

The increase of the survival and persistence may be an increase in stress tolerance of the *L. reuteri* strain and/or an increase in adhesion to mucus of the *L. reuteri* strain. The increase of the probiotic effect may be an increase of the bioavailability of minerals, e.g., by increasing mineral solubility, accessibility and/or absorption of minerals within the gastrointestinal tract of a subject and/or it may also be an increase of epithelial integrity in the gastrointestinal tract and/or an increase in bone formation and/or bone mineralization. The increase of the bioavailability of minerals can in one embodiment result in an increased enzymatic function. As mentioned above, an increased stress tolerance can have positive effects on bacterial survival and/or engraftment and/or viability and/or persistence of the *L. reuteri* strain in the gastrointestinal tract. An increased stress tolerance is preferably an increase of stress tolerance to bile and gastric acid in the intestinal tract of the healthy subject. Any of such effects are typically compared with the effect of a probiotic *L. reuteri* strain not conditioned with GOS.

The increase or boosting of the probiotic effect, typically of said *L. reuteri* strain in the digestive tract of a healthy subject, *inter alia* but not exclusively concerns the increase of the bioavailability of minerals, and specifically the increase of mineral solubility, accessibility and/or absorption of minerals within the gastrointestinal tract of a subject. Such minerals are preferably essential minerals as mentioned herein, e.g., calcium, magnesium and iron.

Of these essential minerals, calcium is involved in conduction of bioelectric impulses, blood coagulation, muscle contraction, prevention of inflammation and hormonal secretion. Magnesium is involved inter alia in neuronal conduction and ribosomal processes. Iron is an important factor in many body functions and processes and is necessary to maintain healthy cells, skin, hair, and nails.

The increase of bioavailability of (such) minerals in the intestinal tract of the healthy subject, preferably by increasing mineral solubility, accessibility and/or absorption in the gastrointestinal tract of the healthy subject thereby preferably allows increasing any of such body functions and processes. These aspects particularly concern non-therapeutical and also cosmetical applications, for which the inventive compositions may be applied.

In addition to such effects on mineral solubility, accessibility and/or absorption of minerals within the gastrointestinal tract of a subject further non-therapeutic uses may encompass an improvement of the *L. reuteri* strain survival and persistence, e.g through an increased stress tolerance and/or increased ability to adhere to mucus in the intestinal tract of a healthy subject, and/or promotion of bone formation and/or bone mineralization, and/or promotion of maturation and/or activity of osteoblasts in a healthy subject, and/or increase of epithelial integrity in the gastrointestinal tract of the healthy subject. Again, such effects are typically to be determined in comparison with the not yet pre-conditioned probiotic L. *reuteri* strain in the digestive tract of a healthy subject.

In this context, a healthy subject, which is the main addressee of this non-therapeutic use, may be selected from an athlete, a child, a toddler or an infant, an adult, an elderly, a pregnant woman, a vegetarian, a lactating woman, a companion animal, a cat, or a dog or a bird, such as poultry, preferably for addressing or even improving such body functions and body processes. Such a healthy subject usually does not suffer from any condition or disease, particularly not from any mineral deficiency as described herein.

Any of the methods as discussed before for pre-conditioning a probiotic *L. reuteri* strain as well as any of the features of compositions as defined herein comprising the pre-conditioned probiotic *L. reuteri* strain apply *mutatis mutandis* for the third aspect of the current invention, namely to the non-therapeutic use of the inventive compositions as described above.

This specifically concerns features of the method of pre-conditioning a probiotic *L. reuteri* strain, and any ingredients and amounts and further features as used for compositions comprising the pre-conditioned probiotic *L. reuteri* strain. The composition may be provided in any form as depicted above.

In this context, it is e.g., noted that the inventive composition for non-therapeutic purposes may be solid or liquid, and/or is present in form of a powder, a tablet, a capsule, or may be in form of an oil formulation, an emulsion, an oil-in-water emulsion (o/w emulsion), or a water-in oil emulsion (w/o emulsion).

Moreover, the inventive composition for non-therapeutic purposes may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains as defined above, preferably selected from group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, more preferably the probiotic *L. reuteri* strain is *L. reuteri* strain as deposited under DSM 17938.

### • USE OF COMPOSITIONS COMPRISING THE PRE-CONDITIONED PROBIOTIC L. REUTERI STRAIN AS A MEDICAMENT

According to the fourth aspect, the invention also provides a composition comprising an effective amount of a pre-conditioned probiotic *L. reuteri* strain as prepared according to the invention or as described herein for use as a medicament. A related aspect defines a pre-conditioned probiotic *L*. *reuteri* strain for use as a medicament.

Similar as before, the effect of such a composition as a medicament is particularly related to the increase of the probiotic effect in the digestive tract of a patient in need of such a medicament, usually compared to the effect of a probiotic *L. reuteri* strain not conditioned with GOS.

The therapeutic effects that are related to the use of the inventive compositions as a medicament *inter* alia address effects related to any of the herein mentioned diseases and applications, particularly in patients that are at risk or that are already suffering from any of such diseases, particularly diseases as mentioned explicitly herein in the context of therapeutic applications (see also below).

Any of the methods as discussed before for pre-conditioning a probiotic *L. reuteri* strain as well as any of the features of compositions as defined herein comprising the pre-conditioned probiotic *L. reuteri* strain also apply *mutatis mutandis* for the fourth aspect of the current invention, namely to the use of the inventive compositions as a medicament.

This specifically concerns features of the method of pre-conditioning probiotic a *L. reuteri* strain, and any ingredients and amounts and further features as used for compositions comprising the pre-conditioned probiotic *L. reuteri* strain. The composition may be provided in any form as depicted above.

In this context, it is particularly preferred that the inventive composition for use as a medicament may be solid or liquid, may be present in form of a powder, a tablet, a capsule, or may be in form of an oil formulation, an emulsion, an oil-in-water emulsion (o/w emulsion), or a water-in oil emulsion (w/o emulsion).

Likewise, the inventive composition for use as a medicament may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains as defined above, preferably selected from group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, more preferably the probiotic *L. reuteri* strain is *L. reuteri* strain as deposited under DSM 17938.

### • THERAPEUTIC USES OF COMPOSITIONS COMPRISING A PRE-CONDITIONED PROBIOTIC L. REUTERI STRAIN

According to a fifth aspect, the invention provides a composition comprising an effective amount of a pre-conditioned probiotic *L. reuteri* strain as prepared according to the invention or as described herein for use in the prevention and/or treatment of a disease in a patient in need thereof or for reducing or inhibiting the risk of such a disease. A related aspect of the invention defines a pre-conditioned probiotic *L. reuteri* strain for use in preventing, reducing or inhibiting, and/or treating a disease in a patient in need thereof, and/or for reducing or inhibiting the risk of such a disease.

As used herein, the terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition, etc. The terms "treatment," "treat" and "to alleviate" are also intended to include the potentiation or otherwise enhancement of one or more primary prophylactic or therapeutic measure. The terms "treatment," "treat" and "to alleviate" are further intended to include the dietary management of a disease or condition or the dietary management for prophylaxis or prevention a disease or condition.

According to one embodiment, the invention is directed to a composition comprising an effective amount of a pre-conditioned probiotic *L. reuteri* strain as prepared according to the inventive method or as defined herein for use in the prevention and/or treatment of mineral deficiency in a patient in need thereof. Typically, a patient in need thereof is also typically a subject that is at risk of developing mineral deficiency or is a subject already suffering from mineral deficiency.

Mineral deficiency in this particular context of the invention preferably concerns a deficiency in calcium, magnesium and/or iron. Thereby, it is not necessarily a deficiency of just one mineral alone that may lead to a specific disease as depicted below but more often a combined lack of different minerals, e.g., one or more minerals selected from calcium, magnesium and/or iron, and, consequently, also an overlap in the physiological consequences and a combination of different diseases/conditions that may occur.

As mentioned before, iron deficiency is a condition, which occurs when the body doesn't have enough of the mineral iron. Iron deficiency often leads to abnormally low levels of red blood cells and a condition referred to as iron deficiency anemia. As a result, iron deficiency anemia results in fatigue, tiredness and shortness of breath. Iron is also known to be very important in maintaining many body functions (such as immune functions or cognitive functions), and is necessary to maintain healthy cells, skin, hair, and nails.

The following groups of people are at highest risk for iron-deficiency anemia and are hence suitable for receiving the pre-conditioned *L. reuteri* strain composition; women who menstruate, particularly if menstrual periods are heavy, women who are pregnant or breastfeeding or those who have recently given birth, people who have undergone major surgery or physical trauma and lost blood, people with gastrointestinal diseases such as celiac disease, inflammatory bowel diseases such as ulcerative colitis, or Crohn's disease, people with peptic ulcer disease, people who have undergone bariatric procedures, especially gastric bypass operations, vegetarians, vegans, and other people whose diets do not include iron-rich foods, children who drink more than 16 to 24 ounces a day of cow's milk (Cow's milk not only contains little iron, but it can also decrease absorption of iron and irritate the intestinal lining causing chronic blood loss), and infants especially those who have low birth weight or are born prematurely, and/or those who don't get enough iron from breast milk or formula. In general, children need extra iron during growth spurts. Any of such patient groups may be treated herein.

Thus, in one embodiment the inventive composition comprising an effective amount of the pre-conditioned probiotic *L. reuteri* strain is as prepared according to the invention or as described herein used to treat iron deficiency, preferably iron deficiency, preferably iron deficiency anemia, in a subject.

Further, chronic iron deficiency is a known emerging risk factor for bone loss and osteoporosis. Calcium is another essential mineral which is important bone component and an activator of enzymes. Calcium also takes part in conduction of bioelectric impulses, blood coagulation, muscle contraction, inflammation and hormonal secretion. Magnesium is a bone component and takes part in neuronal conduction and ribosomal processes.

The inventive composition may also be for use in the prevention and/or treatment of iron deficiency, preferably iron deficiency anemia, and/or promote cognitive functions, and/or promote oxygen transport, and/or promote immune functions, and/or reduces tiredness or fatigue, in a patient in need thereof.

In one embodiment, the present invention therefore provides a composition comprising a pre-conditioned *L. reuteri* strain for use in the prevention and/or treatment of calcium and/or magnesium deficiency in a subject, for use in the prevention and/or the treatment of bone loss, for use in promoting the bone formation and/or mineralization and/or bone strength and/or teeth developments, and/or in promoting growth and development, especially in children and infants, e.g., by promoting the maturation and/or activity of osteoblasts, or for use in the treatment of osteoporosis and/or osteopenia.

Moreover, and particularly in view of calcium deficiencies, further conditions and/or diseases may be prevented or treated related to muscle problems, e.g., cramps, muscle spasms, and aches, fatigue, including insomnia, sleepiness, and extreme fatigue, skin and nail symptoms, eczema, psoriasis, redness, itchiness, skin blisters, osteoporosis and osteopenia, painful premenstrual syndrome, dental problems, depression, etc. Moreover, calcium deficiency (hypocalcemia) is also serious problem in early infancy, which makes infants of said stage to a particular target group. This includes neonatal hypocalcemia, which occurs in the first 2 to 3 days of a baby's life, as well as late hypocalcemia, which starts in the first week or weeks after birth. Also here, low magnesium levels may cause low calcium levels and represent a further risk factor, since calcium levels are linked to a certain extent to levels of magnesium. Any of such patient groups may be treated herein.

Furthermore, particularly in view of magnesium deficiencies, further conditions and/or diseases may be prevented or treated related to tiredness, generalized weakness, muscle cramps, muscular dysfunctions, abnormal heart rhythms, increased irritability of the nervous system with tremors, paresthesia, palpitations, low potassium levels in the blood, hypoparathyroidism resulting in low calcium levels in the blood, chondrocalcinosis, spasticity and tetany, migraines, epileptic seizures, basal ganglia calcifications and in extreme and prolonged cases coma, or intellectual disability. Again, any of such patient groups may be treated herein.

According to a further embodiment the present invention provides a composition comprising a pre-conditioned probiotic *L. reuteri* strain for use in increasing the survival, persistence and/or probiotic effect, preferably of a probiotic *L. reuteri* strain in the intestinal/digestive tract of a subject.

Such an improvement or increase of the survival, persistence and/or probiotic effect may be manifested e.g.:
- as an increase of mineral bioavailability, preferably an increase of mineral solubility and/or mineral absorption, in the intestinal tract of the patient; and/or
- as an increase in stress tolerance of *L. reuteri* strain in the intestinal tract of the patient, preferably an increase of stress tolerance to bile and gastric acid in the intestinal tract of the patient; and/or
- as an increase in adhesion of *L. reuteri* strain to mucus in the intestinal tract of the patient; and/or
- as an increase of epithelial integrity in the intestinal tract of the patient; and/or
- as a promotion of bone formation and/or bone mineralization of the patient; and/or
- a promotion of the maturation and/or activity of osteoblasts of the patient.

Such effects, including e.g. an increase of bioavailability of minerals in the intestinal/digestive tract of a subject, as well as the further effects, is particularly due to the GOS pre-conditioning step of preparing the herein used pre-conditioned *L. reuteri* strain and the observed increased capability of said pre-conditioned *L. reuteri* strain to positively influence and increase bioavailability of minerals in the intestinal tract of the healthy subject, typically by increasing mineral solubility, accessibility and/or absorption of such minerals in the gastrointestinal tract of a subject. Particularly relevant minerals in this context include magnesium, calcium and/or iron, preferably calcium and iron.

As said above, the herein used pre-conditioned probiotic *L. reuteri* strain also positively influences and increases the stress tolerance of the pre-conditioned probiotic *L. reuteri* in the gastrointestinal tract of a patient in need thereof. This has been convincingly demonstrated by the inventors by stress tolerance tests using bile and acidic stress test conditions. Such an improvement of stress tolerance of the pre-conditioned probiotic *L. reuteri* in the gastrointestinal tract of a patient in need thereof leads to an improved capability of the pre-conditioned probiotic *L. reuteri* to better withstand changing bile and acid conditions in the gastrointestinal tract, manifested by an increase of the survival and persistence of the pre-conditioned *L*. *reuteri* strain.

The herein used pre-conditioned *L. reuteri* strain moreover positively influences and increases the adhesion (of the pre-conditioned probiotic *L. reuteri* strain) to mucus. This can have positive effects on the potential for the bacteria to exert its probiotic effects as it would enable them to come closer to the epithelial lining of the intestines and also to remain for longer time periods in the intestine, hence increases its abilities to persist in the gastrointestinal tract.

The herein used pre-conditioned *L. reuteri* strain also increases the epithelial integrity, preferably of the intestinal epithelium in the gastrointestinal tract. It is important in this context to note that the main function of the intestinal barrier is to regulate the absorption of nutrients, electrolytes and water from the lumen into the circulation and, on the other hand, to prevent the passing of microorganisms (e.g., pathogenic microorganisms) and toxic luminal substances into the circulation, an intact barrier is thus essential for obtaining a healthy condition. A disrupted intestinal barrier, or disruptions in other mucosal linings, is associated with several diverse diseases and conditions, such as irritable bowel syndrome (IBS), Crohn's disease, ulcerative colitis, functional gastrointestinal disorders (e.g. functional abdominal pain), diarrhea, infantile colic, infectious diseases, depression, autism spectrum disorders, diverticular disease, periodontitis, osteopenia and/or osteoporosis.

According to one embodiment, the invention is therefore also directed to a composition comprising an effective amount of a pre-conditioned probiotic *L. reuteri* strain as defined prepared according to the inventive method or as defined herein for use in the prevention and/or treatment of a disease in a patient in need thereof, wherein the patient is suffering from a disrupted intestinal barrier, or disruptions in mucosal lining, preferably, wherein the disrupted intestinal barrier, or disruptions in mucosal lining is associated with a disease or condition selected from irritable bowel syndrome (IBS), Crohn's disease, ulcerative colitis, functional gastrointestinal disorders, such as functional abdominal pain, diarrhea, infantile colic, infectious diseases, depression, autism spectrum disorders, diverticular disease, periodontitis, osteopenia and/or osteoporosis.

A further positive effect of the herein used pre-conditioned *L. reuteri* strain is the promotion of bone formation and/or bone mineralization, and/or for promoting the maturation and/or activity of osteoblasts. This has been convincingly demonstrated by the inventors by results on increased ALP enzyme activity in osteoblasts, suggesting that the pre-conditioned *L. reuteri* strain can stimulate osteoblast differentiation and also the enhanced osteocalcin expression which is a marker of mineralization that is expressed by differentiated osteoblasts.

It is therefore submitted that an increase in survival, persistence and/or probiotic effect of the *L. reuteri* strain in the intestinal tract of a patient to be treated may specifically and positively influence the treatment of any of the herein-mentioned diseases. The medical uses as claimed herein therefore also encompass an increase in stress tolerance of a *L. reuteri* strain in the digestive tract of a patient in need thereof, an increase of a *L. reuteri* strain in adhesion to mucus and/or an increase of epithelial integrity in the digestive tract of a patient in need thereof, an increase in mineral solubility and/or mineral accessibility and/or mineral absorption in the digestive tract of a patient in need thereof, and/or increase in bone formation and/or bone mineralization of a patient in need thereof, typically in the context of any of therein mentioned conditions or diseases.

Generally, subject for any such treatments or a patient in need of such a treatment is a mammal or a bird, preferably, it is human, more preferably it is selected form a child, a toddler or an infant, an elderly, a pregnant woman, a vegetarian, a lactating woman, a companion animal, but also companion pets such as a cat or a dog, or a bird, such as poultry, wherein the subject is preferably either at risk of developing mineral deficiency or has already developed a mineral deficiency. Populations at risk for calcium deficiency and preferred subject to such a prevention and/or treatment particularly include pregnant women (especially in the last trimester), lactating women, postmenopausal women, older adults, teenagers, and/or people who are overweight. Populations at risk for hypomagnesemia particularly include diabetes patients, patients at risk of diabetes, obese patients, and/or overweight patients, etc. Any of such subjects and patient groups may be treated herein.

Also, in this context, any of the methods as discussed before for pre-conditioning a probiotic *L. reuteri* strain as well as any of the features of compositions as defined herein comprising the pre-conditioned probiotic *L. reuteri* strain also apply *mutatis mutandis* for the fifth aspect of the current invention, namely to the medical or therapeutic uses of the inventive compositions in any of the herein defined treatments.

This specifically concerns features of the method of pre-conditioning a probiotic *L. reuteri* strain, and any ingredients and amounts and further features as used for compositions comprising the pre-conditioned probiotic *L. reuteri* strain. The composition may be provided in any form as depicted above.

In this context, it is particularly preferred that the inventive composition for medical or therapeutic uses as described herein may be solid or liquid, may be present in form of a powder, a tablet, a capsule, or may be in form of an oil formulation, an emulsion, an oil-in-water emulsion (o/w emulsion), or a water-in oil emulsion (w/o emulsion).

Likewise, the inventive composition for medical or therapeutic uses as described herein may be in an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

As before, the *L. reuteri* strain is particularly preferable at least one *L. reuteri* strain or multiple *L. reuteri* strains as defined above, preferably selected from group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, more preferably the probiotic *L. reuteri* strain is *L. reuteri* strain as deposited under DSM 17938.

According to a further particular embodiment, the current invention also provides a method of treatment of a disease in a patient in need thereof. Such a disease and a patient are preferably as described herein. The method of treatment preferably includes the step of administering to the patient an effective amount of a pre-conditioned probiotic *L. reuteri* strain, preferably as prepared according to the inventive method for pre-conditioning the probiotic *L. reuteri* strain, or as described herein. A pre-conditioned probiotic *L. reuteri* strain is thereby a probiotic *L. reuteri* strain that has been cultivated in the presence of GOS, preferably according to the inventive method for pre-conditioning the probiotic *L. reuteri* strain. Administration allows preferably the prevention and/or treatment of a disease in a patient in need thereof or reducing or inhibiting the risk of such a disease. Any of the diseases as defined above are encompassed. The effective amount of a pre-conditioned probiotic *L. reuteri* strain may be provided in form of an inventive composition as described herein. Any of the features discussed above for the inventive method for pre-conditioning the probiotic *L. reuteri* strain and the inventive composition analogously apply.

According to a another particular embodiment, the current invention also provides the use of a pre-conditioned probiotic *L. reuteri* strain, preferably as prepared according to the inventive method for pre-conditioning the probiotic *L. reuteri* strain, or as described herein for preparing a medicament or pharmaceutical composition for preventing and/or treating a disease in a patient in need thereof. Such a disease and a patient are preferably as described herein. A pre-conditioned probiotic *L. reuteri* strain is thereby a probiotic *L. reuteri* strain that has been cultivated in the presence of GOS, preferably according to the inventive method for pre-conditioning the probiotic *L. reuteri* strain. The use preferably allows the prevention and/or treatment of a disease in a patient in need thereof or reducing or inhibiting the risk of such a disease. Any of the diseases as defined above are encompassed. The effective amount of a pre-conditioned probiotic *L. reuteri* strain may be provided in form of an inventive composition as described herein. Any of the features discussed above for the inventive method for pre-conditioning the probiotic *L. reuteri* strain and the inventive composition analogously apply.

It should be appreciated that the various aspects and embodiments of the detailed description as disclosed herein are illustrative of the specific ways to make and use the invention and do not limit the scope of invention when taken into consideration with the claims and the detailed description. It will also be appreciated that features from aspects and embodiments of the invention may be combined with further features from the same or different aspects and embodiments of the invention.

As used in this detailed description and the appended claims, the singular forms "a," "an" and "the" include plural referents unless the context clearly dictates otherwise.

All ranges described are intended to include all numbers, whole or fractions, contained within the said range.

### EXAMPLES

### Example 1 - Method for pre-conditioning L. reuteri strain with GOS

Internally produced *L. reuteri* strain frozen starter cultures (*L. reuteri* strain DSM 17938) were used to inoculate a fermentation medium containing 4% Bovine milk derived oligosaccharides (BMOS) as carbon source and 4% fructose as electron acceptor (on dry matter). Overnight culture of *L. reuteri* strain was used to inoculate a fresh fermentation medium containing 6% BMOS as carbon source and 6% fructose as electron acceptor (on dry matter). Pre-conditioned *L. reuteri* strain cells were harvested by centrifugation after around 10 h fermentation prior to mixing with protectants and to spray-drying.

BMOS carbohydrate mixture used as source of GOS was composed of 48% GOS, 30% lactose, 8% glucose, 3.9% galactose, and >0.2% 3'-SL + 6'-SL (g/100 g of dry matter).

*L. reuteri* strain cell counts were determined by pour plating. Briefly, serial decimal dilutions spray dried samples were performed in tryptone salt (Oxoid, LP0042) solution. Subsequently, 100 µL of the appropriate dilutions were transferred to petri dishes and mixed with MRS agar (AES Chemunex, Bruz, France). Analysis was performed in duplicate. Plates were then incubated in aerobic conditions at 37°C for 48 h. Colony forming units per gram (cfu/g) were calculated from the number of colonies counted on plates with appropriate dilution by determining their arithmetic mean.

### Example 2 - Method for manufacture of a composition with pre-conditioned L. reuteri strain

A cow milk-based toddler beverage, also called growing-up-milk, containing minerals adapted for the age group was prepared by adding an amount of 5x10⁶ cfu per gram pre-conditioned probiotic *L. reuteri* strain to a conventional commercially available growing-up-milk composition.

### Example 3 - Manufacture of an oil-based formulated probiotic product comprising a pre-conditioned L. reuteri

The *L. reuteri* DSM 17938 strain is cultivated with the addition of GOS according to the method described in Example 7. After cultivation, the pre-conditioned *L. reuteri* DSM 17938 is lyophilized, using standard methods in the industry. The product is an oil-based formulation with a single (pure) bacterial strain made for good stability and shelf life. The unique feature of the production process is a step of drying the oil by placing it under vacuum to remove most of the water in the oil and to increase the stability of the formulation. The oil used herein is a pure edible vegetable oil, preferably sunflower oil and medium-chain triglyceride.

Mixing of ingredients.
1. Mix the medium-chain triglyceride, for example, Akomed R (Karlshamns AB, Karlshamn Sweden), and sunflower oil, for example, Akosun (Karlshamns AB, Karlshamn Sweden), with silicon dioxide (Cab-o-sil M5P, M5P, Cabot) in a Bolz mixing machine/tank (Alfred BOLZ Apparatebau GmbH, Wangen im Allgäu, Germany).
2. Homogenization. A Sine pump and dispax (Sine Pump, Arvada, Colorado) are connected to the Bolz mixer and the mixture is homogenized.
3. Vacuum-drying. The mixture is dried under 10 mBar vacuum in the Bolz tank for 12 hours.
4. Adding the *L. reuteri* DSM 17938 bacteria. About 20 kg of dried oil mixture is moved to a 50 liter stainless steel vessel. *L. reuteri* bacteria powder, preferably freeze-dried; the amount of *L. reuteri* bacteria used would vary depending on the amount wanted in the oil, but one example would be to add 0.2 kg of culture having 10¹¹ CFU per g, is added. It is mixed slowly until homogenous.
5. Mixing. The premix with *L. reuteri* bacteria is brought back to the Bolz mixer.
6. Discharging. The suspension is discharged to a 200-liter glass vessel and covered with nitrogen. The suspension is held in the vessel until filling in bottles to be used for oral administration to a human.

### Example 4 - Manufacture of a probiotic product in a tablet format comprising a pre-conditioned L. reuteri

The *L. reuteri* DSM 17938 strain is cultivated with the addition of GOS according to the method described in Example 7. After cultivation the pre-conditioned *L. reuteri* DSM 17938 is lyophilized, using standard methods in the industry.

The following steps illustrate an example of a manufacturing process for tablets containing the pre-conditioned *L. reuteri* DSM 17938 strain, including glucose encapsulation. It is understood that excipients, fillers, flavors, encapsulators, lubricants, anticaking agents, sweeteners and other components of tablet products as are known in the art, may be used without affecting the efficacy of the product:
1. Melting. Melt SOFTISAN^{™} 154 (SASOL GMBH, Bad Homburg, Germany) in a vessel and heat it to 70°C to assure complete disruption of the crystalline structure. Then cool it down to 52 - 55°C (just above its hardening point).
2. Granulation. Transfer *L. reuteri* strain freeze-dried powder to a Diosna high-shear mixer/granulator, or equivalent. Add slowly during approximately 1 minute the melted SOFTISAN^{™} 154 to the *L. reuteri* strain powder. Use chopper during the addition.
3. Wet-sieving. Immediately after the granulation, pass the granules through a 1 mm sieving net by using a Tornado mill. The sieved granulate is packed in alu-pouches, made out of PVC-coated aluminum foil, sealed with a heat sealer to form a pouch, together with desiccant pouch, and stored refrigerated until mixing. The granulated batch is divided for two tablet batches.
4. Add encapsulated D-glucose (G8270, >99.5 % glucose, Sigma), encapsulated using standard microencapsulating methods as known in the art. The amount of sugar is dependent on the total CFU of the added powder of dry *L. reuteri* strain, a standard level can be 1 gram of sugar per total CFU of 10⁸ of bacteria but this could also be varied down to 0.1 gram or 0.01 gram up to 10 gram even up to 100 gram of sugar.
5. Mixing. Mix all the ingredients in a mixer, to a homogenous blend.
6. Compression. Transfer the final blend to the hopper of a rotary tablet press and compress tablets with a total weight of 765 mg, in a Kilian compressor.
7. Bulk packaging. The tablets are packed in alu-pouches together with a drying pouch of molecular sieve. The alu-pouch is put in a plastic bucket and stored in a cool place at least one week, before final package. The use of SOFTISAN^{™}, a hydrogenated palm oil, enables the *L. reuteri* cells to be encapsulated in fat and environmentally protected.

### Example 5 - Pre-conditioned probiotic L. reuteri strain and increased calcium and magnesium solubility in the colon

### a) SHIME-model

In this experiment, a simplified simulation of the continuous Simulator of the Human Microbial Ecosystem (SHIME^{®}) was used. This SHIME^{®} model has been extensively used for more than 20 years for both scientific and industrial projects and has been validated with *in vivo* parameters (see for example Van den Abbeele et al., Arabinoxylo-Oligosaccharides and Inulin Impact Inter-Individual Variation on Microbial Metabolism and Composition, Which Immunomodulates Human cells, J. Agric. Food chem. 2018, 66, 5, 1121-1130).

For the current set of experiments a two-stage batch system mimicking the upper gastro-intestinal tract (Upper GIT, stomach and small intestine) and colonic conditions were used as simplified SHIME^{®} system (Figure 1). Cow milk-based toddler beverage, also called Growing-up-milk, containing minerals adapted for the age group was used in these studies.

In order to simulate the absorptive processes occurring in the small intestine of toddlers, a dialysis approach was applied by using a cellulose membrane with a cut-off of 14 kDa. By introducing the small intestinal suspension within a dialysis membrane, molecules such as digested amino acids, sugars, micronutrients and minerals were gradually removed from the upper gastro-intestinal matrices.

Furthermore, a gradual pH decrease during the stomach incubation going from 5.5 till 3.0 during 1 h of incubation was implemented to simulate the gastric pH of toddlers. Also, during the first 30 minutes of small intestinal incubation (duodenum), a fixed pH of 4.5 was implemented to allow the available minerals to optimally absorb. The following 145 minutes of the small intestinal phase (jejunum + ileum), a pH of 7 was introduced. The milk matrix after exposure to gastric and small intestinal conditions was transferred to the colonic compartment containing the fecal sample of a toddler.

Fresh fecal material was collected from a 12-month-old infant donor. Fecal suspension was prepared and mixed with a protectant. At the start of the short-term colonic incubation, the test ingredients (*L. reuteri* strain and pre-conditioned *L. reuteri* strain in accordance with Example 1) were added to sugar-depleted nutritional medium containing basal nutrients present in the colon (e.g., host-derived glycans such as mucin).

*L. reuteri* strain and pre-conditioned *L. reuteri* strain DSM 17938 were inoculated at 1E+09 CFU/reactor.

Following colonic incubations were performed:
- Control incubation without probiotic (Upper-GIT digested milk alone)
- Upper GIT digested milk +*L*. *reuteri* strain DSM 17938
- Upper GIT digested milk + Pre-conditioned *L. reuteri* strain DSM 17938

Colonic incubations were performed during 48 h, at 37°C and under shaking (90 rpm) conditions. All experiments were performed in triplicate to account for biological variation.

### b) Analysis of minerals (calcium and magnesium):

Analysis of calcium and magnesium was performed by a specialized laboratory of Ghent University, Belgium. The samples were measured by ICP-OES. Samples for mineral analysis in the colon compartment were taken after 0 h, 24 h and 48 h. The 48 h timepoint was preceded by a colonic dialysis, resulting in values for the bio-accessible mineral fraction and the insoluble mineral fraction.

As can be seen in Figure 2, pre-conditioned probiotic *L. reuteri* strain boosts magnesium and calcium bio-accessibility in colon at both 24 h and 48 h. This suggests that a pre-conditioned *L. reuteri* strain can be used to promote magnesium and calcium absorption in a subject in need thereof.

### Example 6 - Pre-conditioned L. reuteri strain and increased iron bio-accessibility in the colon

The experiment was carried out as described in Example 5. Similarly, analysis of iron contents was performed by a specialized laboratory of Ghent University, Belgium. The samples were measured by ICP-OES. Samples for mineral analysis in the colon compartment were taken after 0 h, 24 h and 48 h. The 48 h timepoint was preceded by a colonic dialysis, resulting in values for the bio-accessible mineral fraction and the insoluble mineral fraction.

As can be seen in Figure 3, the pre-conditioned probiotic *L. reuteri* strain boosts iron bio-accessibility in colon. This suggests that a pre-conditioned *L. reuteri* strain can be used to promote iron absorption in a subject in need thereof.

### Example 7 - Pre-conditioned L. reuteri strain improves stress tolerance to gastrointestinal conditions

For this experiment, *L. reuteri* strain DSM 17938 was cultivated with the addition of different sugars and electron acceptors (i.e. 3% BMOS + 3% fructose or 3% glucose + 3% fructose) before the bacteria were lyophilized until further testing with either of the two stress assays described below; acid tolerance (c)) or bile tolerance (d)). Results are presented under e).

### a) Cultivation in bioreactor

Ten ml MRS media (Oxoid) were inoculated with 100 µl from the frozen stock stored at -70°C. The tube was incubated for 6 h at 37 °C and thereafter 500 µl was used for inoculation of a tube with 50 ml MRS. The tube was incubated at 37 °C for 16-20 hours and thereafter the whole volume was used as inoculum to the bioreactor.

Conditions for cultivation in bioreactor:
Working volume: 1 L
Medium: iMRS* with 3% BMOS+3% fructose or 3% glucose + 3% fructose
Aeration: No aeration
Temperature: 37 °C
Stirring: 250 rpm
pH: 6.00 +/- 0.1
Cultivation time: 6-10 h

### *iMRS ingredients

| Component | Quantity(g) |
|---|---|
| Yeast extract | 10 |
| Yeast peptone | 10 |
| Sodium citrate | 2 |
| Sodium acetate | 5 |
| K₂HPO₄ | 2 |
| MgSO₄ heptahydrate | 0.1 |
| Manganese sulfate heptahydrate | 0.05 |
| Tween 80 | 1 |
| L-cysteine | 0.1 |
| Purified water (800 ml) | |
| Set pH to 6,5 with NaOH | |

Autoclave (121 °C 15 min) and thereafter add sterile sugar solution (final volume 1000 ml)

After cultivation, the bacteria were pelleted by centrifugation and suspended in 50 ml 10% sucrose.

### b) Lyophilization

The concentrated cell suspensions were dispensed into freeze-drying glass vials (1 ml/vial) and the vials were frozen in an ultra-freezer at -50 °C for 2 h before being transferred to a Labconco FreeZone Stoppering Tray Freeze-Dryer. The freeze-drying scheme was set-up as follows: Freezing step: -40 °C for 4h; Main drying: -35 °C for 15 min at 0.102 mbar, -10 °C for 12.5 h at 0.102 mbar; secondary drying: -10 °C for 30 min at 0.01 mbar, 25 °C for 12 h at 0.01 mbar. At the end of the process the vials were capped under vacuum and stored at -20 °C until further use.

### c) Stress tolerance Acid - assay

One vial of cultivated and lyophilized *L. reuteri* strain DSM 17938 was rehydrated in 1 ml PBS (pH 7.4), and thereafter diluted 20 times in 10 ml synthetic gastric juice pH 2,0* or PBS (control). The tubes were incubated at 37°C, samples taken out at 0, 50 min, 90 min, serially diluted (10⁻¹ - 10⁻⁶) and plated on MRS agar plates. The plates were incubated in anaerobic jars at 37°C overnight before counting the colonies.
* Synthetic gastric juice pH 2,0
8.3 g/l proteose peptone (Oxoid)
3.5 g/l D-glucose
2.05 g/l NaCl
0.6 g/l KH₂PO₄
0.11 g/l CaCl₂
0.37 g/l KCI
0.05 g/l bile
0.1 g/l lysozyme (add before use)
13,3 mg/l pepsin (add before use)
Adjust the pH to 2.0 using 1M HCI
Filter sterilize the juice.

### d) Stress tolerance 8% bovine bile - assay

One vial of cultivated and lyophilized *L. reuteri* strain DSM 17938 cells was rehydrated in 1 ml PBS (pH 7.4), thereafter diluted 20 times in 10 ml MRS (Oxoid) with 8% bovine bile or PBS (control). The tubes were incubated at 37°C, whereafter samples were taken out at 0, 30 min, 90 min, serially diluted (10⁻¹ - 10⁻⁶) and plated on MRS agar plates. The plates were incubated in anaerobic jars at 37°C overnight before counting the colonies.

### e) Results

The results from c) and d) show that pre-conditioned *L. reuteri* strain DSM 17938 (i.e. cultivated in 3% BMOS + 3% fructose) has improved stress tolerance to both acid and bile compared to *L. reuteri* strain DSM 17938 cultivated in 3% glucose + 3% fructose (*L. reuteri* strain DSM 17938) in all assays, see Figure 4 and 5. Bacteria with increased stress tolerance to both acid and bile, as shown here in two separate tests, will perform better in the gastrointestinal tract of a subject such as having an improved survival and persistence.

### Example 8 Pre-conditioned L. reuteri strain improves adhesion to mucus

For this experiment, *L. reuteri* strain DSM 17938 was cultivated in a bioreactor with the addition of different sugars and electron acceptors before the bacteria were lyophilized according to the procedures described in Example 7 above. The *L. reuteri* strain DSM 17938 was then evaluated for its adhesion properties as described below.

### Material and methods

### a) Coating of microtiter wells with mucus

Mucus was prepared from the small intestine of a pig. The inside of 5 cm intestine was scraped with a spatula and material removed and collected in 5 ml ice-cold PBS. The resulting suspension was centrifuged first at 11,000 x g for 10 min and then at 26,000 x g for 15 min in order to remove cells and particulate matter. The mucus preparation was stored at - 20°C until used.

The mucus material was diluted to OD₂₈₀ = 0.1 in PBS pH 6 and incubated overnight in microtiter wells (Greiner or Nunc MaxiSorb) (150 µl per well) at 4°C with slow agitation. The wells are thereafter washed with PBS (pH 6.0) with 0.05% Tween 20 (PBST), thereafter blocked with 0.2 ml PBS with 1% Tween 20 (pH 6.0) for 1 h and finally washed twice with PBST.

### b) Preparation of the bacteria

One vial of cultivated and lyophilized *L. reuteri* strain DSM 17938 cells was rehydrated in 1 ml PBS (pH 7.4) and washed with PBS 7.4 +Tween 20 (0.05%). The samples were diluted to OD 0.5 and thereafter washed twice with PBST 7.4 (1 ml) and finally resuspended in PBST pH 6.0. The number of cfu/ml were analyzed by diluting the suspensions in MRS broth and thereafter plating the bacteria on MRS agar plates (Merck) which were incubated at 37°C at 48 h under anaerobic conditions.

### c) Binding

Bacterial suspensions (150 µl) were added to each well and incubated for 4 h at 37°C with slow agitation. After 4 hours of binding, wells were washed 3 times with PBST (0.05% Tween 20 pH 6.0) and treated with 150 µL trypsin EDTA solution (0.25% porcine trypsin and 1 mM EDTA • 4Na in Hanks' Balanced Salt Solution) for 30 min at 37°C and the bacterial suspensions were thereafter serially diluted and plated on MRS plates. The plates were incubated under anaerobic conditions for 24 h at 37°C.

### d) Results

Pre-conditioned *L. reuteri* strain DSM 17938 (i.e. cultivated in 3% BMOS + 3% fructose) has improved mucus binding and adhesion capabilities as compared to *L. reuteri* strain DSM 17938 cultivated in 3% glucose + 3% fructose (*L. reuteri* strain DSM 17938), see table 1 below.

The results indicate that pre-conditioned *L. reuteri* strain DSM 17938 would have improved abilities of attaching to the intestinal mucosa as compared to *L. reuteri* strain DSM 17938. This can have positive effects on the potential for the bacteria to exert its probiotic effects as it would enable them to come closer to the epithelial lining of the intestines and also to remain for longer time periods in the intestine.

**Table 1. Mucus binding capabilities.**

| **Samples** | **Binding %** |
|---|---|
| Pre-conditioned *L. reuteri* | 0.5 |
| *L. reuteri* | 0.003 |

Example 9 Pre-conditioned *L. reuteri* strain protects the epithelial integrity

For this experiment, *L. reuteri* strain DSM 17938 was cultivated in bioreactor with the addition of different sugars and electron acceptors before the bacteria was lyophilized according to the procedures described in Example 7 above. The *L. reuteri* strain DSM 17938 was then evaluated for its capabilities of protecting the intestinal epithelium from detrimental effects of enterotoxigenic *Escherichia coli* (ETEC) as described below.

### Material and Methods

### Epithelial cell culture (Caco-2/HT29 MTX)

Caco-2 and HT29 MTX cells were separately grown in tissue culture flasks in Dulbecco's Modified Eagle's Medium (DMEM) supplemented with 10% fetal bovine serum, 1% non-essential amino acids, and 1% penicillin and streptomycin, at 37°C under an atmosphere of 5% CO₂ with 90% relative humidity. Caco-2 and HT29-MTX cells were grown in 25 cm² tissue culture flasks and split at 80-90% confluence using 0.25% trypsin and 0.02% Ethylenediaminetetraacetic acid (EDTA) solution. The cells were seeded at a density of 6x10⁴ cells per 25-cm² flask.

### Cell co-cultures

Caco-2 and HT29-MTX cells were seeded on the apical chamber of Transwell inserts (Transwell-COL collagen-coated membrane filters) with 9:1 proportion and grown in 12-well Transwell plates with a final density of 1x10⁵ cells/cm² in each insert. Cells were maintained in the same conditions and allowed to grow for 21 days with medium (0.5 ml on the apical side and 1.5 ml on the basolateral side). Change of medium every other day allowed the cells to become differentiated.

### The integrity of the cell layer

The integrity of the cell layer was determined using two methods: Transepithelial electrical resistance (TEER) and determination of fluorescein isothiocyanate-dextran (FITC-dextran) permeability.

The cell monolayer integrity during the experiments was determined by Transepithelial electrical resistance (TEER) measurement using the Millicell electrical resistance system (Millipore, Darmstadt, Germany). Three different areas were used to detect the TEER values in each well (used the average). Wells with TEER values above 250 Ω cm² were used for the permeability studies.

Seeded Caco-2/HT29-MTX cells were pre-treated with rehydrated *L. reuteri* strain DSM 17938 bacterial cells (cultivated with different carbon sources and lyophilized according to the cultivation and lyophilization procedure above) using PBS (pH 7,4) at 100 multiplicity of bacteria (MOB) for 6 h before challenge with ETEC (enterotoxigenic *E. coli,* known for having a disruptive effect on epithelial integrity) at 100 multiplicity of infection (MOI) for an additional 6 h. ETEC (strain 853/67) was inoculated from a frozen stock stored at -70°C and grown in 10 ml LB broth (Miller's) for 16 h at 37°C. Then, 1 ml of the ETEC overnight culture was re-inoculated into 100 ml LB broth (Miller's) for 4-6 h at 37°C. TEER was measured prior to pre-treatment and challenge with ETEC, followed by measurement every second hour during the entire challenge. In order to quantify the paracellular permeability of monolayers, 1 mg/mL of 4 kDa fluorescein isothiocyanate-dextran (FITC-dextran; Sigma) was added to the apical side of the inserts at the start of the challenge with ETEC. Samples from the basolateral compartment were taken after 6 h of incubation. The diffused fluorescent tracer was then analyzed in triplicate by fluorometry (excitation, 485 nm; emission, 520 nm) using a FLUOstar Omega Microplate Reader (BMG Labtech, Ortenberg, Germany).

### Results

Pre-conditioned *L. reuteri* strain DSM 17938 (cultivated in 3% BMOS + 3% fructose) was found to have improved capability of protecting the epithelial barrier integrity from the detrimental effect of ETEC, compared to *L. reuteri* strain DSM 17938 cultivated in 3% glucose + 3% fructose (*L. reuteri* strain DSM 17938), see Figure 6.

These results indicate that the pre-conditioning of *L. reuteri* strain DSM 17938 improves its protective effect on the barrier integrity.

### Example 10 - Effect on bones

*In vitro* experiments were carried out using a murine pre-osteoblast cell model: MC3T3-E1 (osteoblast are the bone forming cells). Cells were exposed to different conditions: upper GIT-digested milks supplemented with *L. reuteri* samples (with and without pre-conditioning) after 48 h colonic incubations were centrifuged and filtered before being added at 1% in the culture medium (DMEM):

Conditions:
- Digested milk (control)
- Digested milk *+ L. reuteri* strain
- Digested milk + preconditioned *L. reuteri* strain

Read outs:
- APL activity (enzymatic activity). APL enzyme is expressed by Osteoblasts cells when mature. Hence this is an indicator of differentiation of bone forming cells (osteoblasts).
- Osteocalcin gene expression is an indicator of osteoblast mineralization.

As can be seen in Figure 7, a positive effect on osteoblast proliferation with a significant superiority of pre-conditioned *L. reuteri* strain compared to *L. reuteri* strain vs control condition has been observed. Impact on osteoblast differentiation may be associated with a positive impact on bone formation rate thus speed of growth.

As shown in Figure 8, mineralization (late osteoblast activity) is higher with pre-conditioned *L. reuteri* strain. Higher mineralization may lead to increased bone strength.

## Claims

1. A method for preparing a pre-conditioned probiotic *Lactobacillus reuteri* strain **characterized in that** the method comprises the following steps:
a) cultivating a probiotic *L. reuteri* strain in the presence of galacto-oligosaccharides (GOS) in a growth medium, thereby pre-conditioning the probiotic *L. reuteri* strain; and
b) harvesting the pre-conditioned probiotic *L. reuteri* strain from the growth medium.

2. The method according to claim 1, wherein GOS is added in step a) to the growth medium in an amount of at least 0.2 wt%, preferably at least 0.5 wt%, even more preferably at least 0.75 wt% of GOS in the growth medium, and preferably at most 8 wt% of GOS, such as at most 7 wt% GOS, at most 6 wt% GOS, or even at most 5 wt% GOS, more preferably at most 4 wt% of GOS or even at most 3% GOS, such as in a range of 0.2 wt% to 8 wt% or even 0.2 wt% to 7 wt%, or 0.2 wt% to 6 wt%, more preferably in a range of 0.5 wt% to 8 wt% or even 0.5 wt% to 7 wt%, or 0.5 wt% to 6 wt%, even more preferably in a range of 0.75 wt% to 8 wt% or even 0.75 wt% to 7 wt%, or 0.75 wt% to 6 wt%, and most preferably in a range of 0.75 wt% to 7 wt%, in a range of 0.75 wt% to 6 wt% or even in a range of 0.75 wt% to 5 wt%, such as a range of 0.75 wt% to 3 wt% or 0.75 wt% to 4 wt%, preferably calculated on basis of the growth medium (% w/w) preferably at the start, in the middle or at the end of cultivation of the probiotic *L. reuteri* strain in step a).

3. The method according to claim 1 or 2, wherein GOS added to the growth medium in step a) has a degree of polymerization (DP) of 3-8.

4. The method according to claim any of claims 1 to 3, wherein the probiotic *L. reuteri* strain is at least one *L. reuteri* strain or multiple *L. reuteri* strains selected from the group comprising or consisting *of L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.

5. A composition comprising an effective amount of a pre-conditioned probiotic *Lactobacillus reuteri* strain, wherein the pre-conditioned probiotic *L. reuteri* strain has been prepared by cultivating a *L. reuteri* strain in the presence of galacto-oligosaccharides (GOS) in a growth medium, preferably according to a method according to any of claims 1 to 4.

6. The composition according to claim 5, wherein the composition comprises the pre-conditioned probiotic *L. reuteri* strain in an amount of between 10³ cfu to 10¹² cfu, typically in an amount of between 10⁴ cfu to 10¹¹ cfu per daily dose, preferably in an amount of between 10⁵ cfu to 10¹⁰ cfu per daily dose, or 10⁵ cfu to 10⁹ cfu per daily dose, likewise preferably in an amount of between 10⁶ cfu to 10⁹ cfu per daily dose, 10⁶ cfu to 10⁸ cfu per daily dose or in an amount of 10⁸ cfu to 10¹⁰ cfu per daily dose, more preferably around 10⁷ cfu to 10⁹ cfu per daily dose, most preferably in an amount of around 10⁸ total cfu per daily dose, such as 10⁷ to 10⁹ cfu per daily dose or 10⁸ to 10⁹ cfu per daily dose.

7. The composition according to any of claims 5 to 6, wherein
the composition is solid or liquid, and/or is present in form of a powder, a tablet, a capsule, or may be in form of an oil formulation, an emulsion, an oil-in-water emulsion (o/w emulsion), or a water-in oil emulsion (w/o emulsion); and/or
the composition is an administrable form, preferably selected from the group consisting of nutritional compositions, pharmaceutical formulations, dietary supplements, functional food products, functional beverage products, and combinations thereof, e.g., a dairy product, a milk-based product, a whey protein-based beverage.

8. Non-therapeutic use of an effective amount of a pre-conditioned probiotic *L. reuteri* strain as prepared according to a method of any of claims 1 to 4 or a composition according to any of claims 5 to 7 for improving or increasing the survival, persistence and/or probiotic effect, preferably of a *L. reuteri* strain, in the digestive tract of a healthy subject.

9. The non-therapeutic use according to claim 8, wherein improving or increasing the survival, persistence and/or probiotic effect:
- is an increase of mineral bioavailability, preferably an increase of mineral solubility and/or mineral absorption, in the intestinal tract of the healthy subject, wherein the mineral is optionally selected from magnesium, calcium and/or iron, preferably calcium and/or iron; and/or
- is an increase in stress tolerance of *L. reuteri* strain in the intestinal tract of the healthy subject, preferably an increase of stress tolerance to bile and gastric acid in the intestinal tract of the healthy subject; and/or
- is an increase in adhesion of *L. reuteri* strain to mucus in the intestinal tract of the healthy subject; and/or
- is an increase of epithelial integrity in the intestinal tract of the healthy subject; and/or
- is a promotion of bone formation and/or bone mineralization, and/or a promotion of the maturation and/or activity of osteoblasts of the healthy subject.

10. A composition comprising an effective amount of a pre-conditioned probiotic *L. reuteri* strain as prepared according to any of claims 1 to 4 or a composition according to any of claims 5 to 7 for use as a medicament.

11. A composition comprising an effective amount of a pre-conditioned probiotic *L. reuteri* strain as prepared according to any of claims 1 to 4 or a composition according to any of claims 5 to 7 for use in the prevention and/or treatment of a disease in a patient in need thereof, wherein the patient is suffering from a disrupted intestinal barrier, or disruptions in mucosal lining, preferably, wherein the disrupted intestinal barrier, or disruptions in mucosal lining is associated with a disease or condition selected from irritable bowel syndrome (IBS), Crohn's disease, ulcerative colitis, functional gastrointestinal disorders, such as functional abdominal pain, diarrhea, infantile colic, infectious diseases, depression, autism spectrum disorders, diverticular disease, periodontitis, osteopenia and/or osteoporosis.

12. A composition comprising an effective amount of a pre-conditioned probiotic *L. reuteri* strain as prepared according to any of claims 1 to 4 or a composition according to any of claims 5 to 7 for use in the prevention and/or treatment of mineral deficiency in a patient in need thereof, wherein the mineral is optionally selected from magnesium, calcium and/or iron, preferably calcium and/or iron.

13. The composition for use according to claim 12, wherein
the composition is for use in the prevention and/or treatment of iron deficiency, preferably iron deficiency anemia, and/or promote cognitive functions, and/or promote oxygen transport, and/or promote immune functions, and/or reduces tiredness or fatigue, in a patient in need thereof; and/or
the composition is for use in the prevention and/or treatment of calcium and/or magnesium deficiency in a patient in need thereof.

14. A composition comprising an effective amount of a pre-conditioned probiotic *L. reuteri* strain as prepared according to any of claims 1 to 4 or a composition according to any of claims 5 to 7 for use in the prevention and/or the treatment of bone loss, and/or to promote growth and development of bones or teeth in children, and/or promote muscular functions, and/or promote functions of the nervous system and/or bone or muscular related conditions in a patient in need thereof, and/or for use in promoting the bone formation and/or bone mineralization and/or bone strength in a patient in need thereof.

15. The composition for use according to any of claims 10 to 14, wherein the *L. reuteri* strain is at least one *L. reuteri* strain or multiple *L. reuteri* strains selected from the group comprising or consisting of *L. reuteri* strain as deposited under DSM 17938, *L. reuteri* strain as deposited under ATCC PTA 5289, *L. reuteri* strain as deposited under ATCC PTA 6475, *L. reuteri* as deposited under DSM 32846, *L. reuteri* as deposited under DSM 32848, *L. reuteri* as deposited under DSM 32849, *L. reuteri* as deposited under DSM 27131, *L. reuteri* as deposited under DSM 32465 and *L. reuteri* strain as deposited under ATCC PTA 4659, preferably the probiotic *L. reuteri* strain is *L. reuteri* strain as deposited under DSM 17938.
